# EUROPEAN PATENT SPECIFICATION

(11) **EP 2 539 436 B1**
(45) Date of publication and mention of the grant of the patent: **22.09.2021**
(21) Application number: 11748062.4
(22) Date of filing: 24.02.2011
(51) Int. Cl.: C12N 5/074, C12N 5/00, A61K 35/12, A61P 29/00

(54) **MODULATION OF MACROPHAGE ACTIVATION**
MODULIERUNG EINER MAKROPHAGEN-AKTIVIERUNG
MODULATION D'ACTIVATION DE MACROPHAGES

(30) Priority: 25.02.2010 US 308082 P; 23.03.2010 US 316647 P
(43) Date of publication of application: 02.01.2013
(60) Divisional application: 21175415.5
(73) Proprietor: ABT Holding Company, Cleveland, OH 44115 (US); Case Western Reserve University, Cleveland, OH 44106 (US)
(72) Inventor: TING, Anthony, E., Shaker Heights, OH 44122 (US); MAYS, Robert, W., Shaker Heights, OH 44120 (US); HAMILTON, Jason, A., Shaker Heights, OH 44120 (US); BUSCH, Sarah, A., Cleveland, OH 44120 (US); SILVER, Jerry, Bay Village, OH 44140 (US)
(74) Representative: Cornish, Kristina Victoria Joy
(86) International application number: PCT/US2011/026067
(87) International publication number: WO 2011/106521

(56) References cited:
- WO-A1-2010/028249
- US-A1- 2008 194 637
- US-A1- 2008 213 227
- US-A1- 2008 286 246
- US-B1- 7 015 037
- "Posters", GLIA, vol. 57, no. S13, 24 August 2009 (2009-08-24), pages S26-S171, XP055068951, ISSN: 0894-1491, DOI: 10.1002/glia.20915
- R Perry ET AL: "Clinical Scale Expansion of Human Pluripotent Stem Cells", Blood (ASH Annual Meeting Abstracts) 2005 106: Abstract 1060, 1 January 2005 (2005-01-01), XP055069164, Retrieved from the Internet: URL:http://abstracts.hematologylibrary.org /cgi/content/abstract/106/11/1060?maxtosho w=&hits=10&RESULTFORMAT=&fulltext=Clinical +scale+expansion+of+human+pluripotent+stem +cells&searchid=1&FIRSTINDEX=0&volume=106& issue=11&resourcetype=HWCIT [retrieved on 2013-07-02]
- BUSCH SARAH A ET AL: "Overcoming macrophage-mediated axonal dieback following CNS injury", JOURNAL OF NEUROSCIENCE, SOCIETY FOR NEUROSCIENCE, US, vol. 29, no. 32, 12 August 2009 (2009-08-12), pages 9967-9976, XP009127714, ISSN: 0270-6474, DOI: 10.1523/JNEUROSCI.1151-09.2009
- KIM J ET AL: "Mesenchymal stem cell-educated macrophages: A novel type of alternatively activated macrophages", EXPERIMENTAL HEMATOLOGY, ELSEVIER INC, US, vol. 37, no. 12 1 December 2009 (2009-12-01), pages 1445-1453, XP026756830, ISSN: 0301-472X, DOI: 10.1016/J.EXPHEM.2009.09.004 [retrieved on 2009-09-20]
- SIMONSEN J L ET AL: "Telomerase expression extends the proliferactive life-span and maintains the osteogenic potential of human bone marrow stromal cells", NATURE BIOTECHNOLOGY, GALE GROUP INC, NEW YORK, vol. 20, 1 June 2002 (2002-06-01), pages 592-596, XP002902749, ISSN: 1087-0156, DOI: 10.1038/NBT0602-592
- S ZIMMERMANN ET AL: "Lack of telomerase activity in human mesenchymal stem cells", LEUKEMIA, vol. 17, no. 6, 1 June 2003 (2003-06-01), pages 1146-1149, XP055061518, ISSN: 0887-6924, DOI: 10.1038/sj.leu.2402962
- YASER ATLASI ET AL: "OCT4 Spliced Variants Are Differentially Expressed in Human Pluripotent and Nonpluripotent Cells : OCT4 SPLICED VARIANTS ARE DIFFERENTIALLY EXPRESSED IN HUMAN PLURIPOTENT AND NONPLURIPOTENT CELLS", STEM CELLS (MIAMISBURG), vol. 26, no. 12, 11 September 2008 (2008-09-11), pages 3068-3074, XP055667824, ISSN: 1066-5099, DOI: 10.1634/stemcells.2008-0530
- TAKEDA J ET AL: "Human Oct3 gene family: cDNA sequences, alternative splicing, gene organization, chromosomal location and expression at low levels in adult tissues", NUCLEIC ACIDS RESEARCH ADVANCE ACCESS, OXFORD UNIVERSITY PRESS, GB, vol. 20, no. 17, 1 September 1992 (1992-09-01), pages 4613-4620, XP002995407, ISSN: 0305-1048
- "Kuby Immunology 6th edition", 2006
- DATABASE MEDLINE [Online] US NATIONAL LIBRARY OF MEDICINE (NLM), BETHESDA, MD, US; November 1990 (1990-11), MURRAY D W ET AL: "Macrophages stimulate bone resorption when they phagocytose particles.", Database accession no. NLM2246303 & MURRAY D W ET AL: "Macrophages stimulate bone resorption when they phagocytose particles.", THE JOURNAL OF BONE AND JOINT SURGERY. BRITISH VOLUME NOV 1990, vol. 72, no. 6, November 1990 (1990-11), pages 988-992, ISSN: 0301-620X
- DATABASE MEDLINE [Online] US NATIONAL LIBRARY OF MEDICINE (NLM), BETHESDA, MD, US; 1 January 2008 (2008-01-01), MARTINEZ FERNANDO ONEISSI ET AL: "Macrophage activation and polarization.", Database accession no. NLM17981560 & MARTINEZ FERNANDO ONEISSI ET AL: "Macrophage activation and polarization.", FRONTIERS IN BIOSCIENCE : A JOURNAL AND VIRTUAL LIBRARY 01 JAN 2008, vol. 13, 1 January 2008 (2008-01-01), pages 453-461, ISSN: 1093-9946
- CAVAILLON, JM: "Cytokines and macrophages", BIOMED & PHARMACOTHER, vol. 48, 1994, pages 445-453,
- CHARLES A. DINARELLO: "Proinflammatory Cytokines", CHEST, vol. 118, 2000, pages 503-508,
- "The detection, measurement, and characterization of antibodies and their use as research and diagnostic tools" In: Kenneth Murphy ET AL: "Janeway's IMMUNOBIOLOGY, 7TH EDITION", 2006, Garland Science pages 741-743, 753-756,
- KRISTINA A. KIGERL ET AL: "Identification of Two DistinctMacrophage Subsets withDivergent Effects Causing either Neurotoxicity orRegeneration in the InjuredMouse Spinal Cord", THE JOURNAL OF NEUROSCIENCE, vol. 29, 2009, pages 13435-13444,

## Description

### FIELD OF THE INVENTION

The invention provides a method for determining an effective amount of cells for treating conditions associated with an undesirable inflammatory component, e.g., conditions of the central nervous system (CNS). The invention is generally directed to reducing inflammation by administering cells that modulate macrophage activation. The invention is also directed to drug discovery methods to screen for agents that modulate the ability of the cells to modulate macrophage activation. The invention is also directed to cell banks that can be used to provide cells for administration to a subject, the banks comprising cells having a desired potency for modulating macrophage activation. The invention is also directed to compositions comprising cells of specific potency for modulating macrophage activation, such as pharmaceutical compositions. The invention is also directed to methods for evaluating the dose efficacy of the cells in a patient by assessing the *in vitro* activation of macrophages. The invention is also directed to diagnostic methods conducted prior to administering the cells to a subject to be treated, including assays to assess the desired potency of the cells to be administered. The cells are non-embryonic stem, non-germ cells that can be characterized by one or more of the following: extended replication in culture and express markers of extended replication, such as telomerase, markers of pluripotentiality, and broad differentiation potential, without being transformed.

Document "Posters", Glia, Vol 57, no 13, 2009, page 223, starting on page 579 discloses that Multistem ®prevents macrophage-mediated axonal dieback following spinal cord injury.

### SUMMARY OF THE INVENTION The invention is as set out in the claims.

The invention is broadly directed to determination, array and construction methods for cells which achieve immunomodulation by modulating macrophage activation.

The invention is more specifically directed to the above methods for immunomodulation within the CNS by modulating macrophage activation.

The invention is also more specifically directed to the above methods for reducing macrophage neurotoxic activation and/or increasing macrophage neuroprotective activation in CNS conditions.

The invention is also more specifically directed to the above methods for reducing antigen presentation by macrophages. Such reduction may result, for example, from reduced expression of one or more genes involved in the antigen presentation.

The invention is also more specifically directed to the above methods for modulating the activation state of macrophages by means of secreted factors.

The invention is also more specifically directed to the above methods for modulating the activation state of macrophages in CNS conditions by secreted factors that increase neuroprotective activation and/or reduce neurotoxic activation.

The invention is also directed to the above methods for driving macrophages towards a TH2 (neuroprotective) immune response or away from a TH1 (neurotoxic) immune response in CNS injury.

The invention is directed to methods to determine cells for the administration of cells for reducing CNS injury, including, but not limited to, stoke, ischemic stroke, multiple sclerosis, Alzheimer's Disease, ALS, Parkinson's Disease, hypoxic-ischemia, neonatal hypoxic ischemia, and traumatic brain or spinal cord injury, by modulating macrophage activation.

The invention is also directed to methods to determine cells for reducing CNS injury by increasing neuroprotective activation by macrophages and/or reducing neurotoxic activation.

Factors that induce neuroprotective activation include, but are not limited to, CCL21 and CXCL10. Factors that suppress neurotoxic activation include, but are not limited to, TGFβ, CCL5, NGF, Galectin-1, Pentraxin-3, TGF-β, VEGF, BDNF, HGF, adrenomedullin, and thrombospondin.

Factors secreted by macrophages during activation include, but are not limited to, iNOS, CD16, CD86, CD64, and CD32, scavenger receptor A, CD163, arginase 1, CD14, CD206, CD23, and scavenger receptor B.

Factors secreted by macrophages during neurotoxic activation include, but are not limited to, iNOS, CD16, CD86, CD64, and CD32. Factors secreted by macrophages during neuroprotective activation include, but are not limited to, scavenger receptor A, CD163, arginase 1, CD14, CD206, CD23, and scavenger receptor B.

According to this invention, providing the above effects can be achieved by administering cells naturally (i.e., non-recombinantly) expressing and/or secreting one or more modulatory factors or medium conditioned by the cells. Cells are not embryonic stem cells and not germ cells, having some characteristics of embryonic stem cells, but being derived from non-embryonic tissue, and expressing and/or secreting one or more modulatory factors as set out in the claims. The cells are multipotent adult progenitor cells (MAPCs). The cells may naturally express/secrete one or more modulatory factors (i.e., not genetically or pharmaceutically modified to activate expression and/or secretion). However, natural expressors can be genetically or pharmaceutically modified to increase potency.

The cells may express pluripotency markers, such as oct4. They may also express markers associated with extended replicative capacity, such as telomerase. Other characteristics of pluripotency can include the ability to differentiate into cell types of more than one germ layer, such as two or three of ectodermal, endodermal, and mesodermal embryonic germ layers. Such cells may or may not be immortalized or transformed in culture. The cells may be highly expanded without being transformed and also maintain a normal karyotype. For example, in one embodiment, the non-embryonic stem, non-germ cells may have undergone at least 10-40 cell doublings in culture, such as 50, 60, or more, wherein the cells are not transformed and have a normal karyotype. The cells may differentiate into at least one cell type of each of two of the endodermal, ectodermal, and mesodermal embryonic lineages and may include differentiation into all three. Further, the cells may not be tumorigenic, such as not producing teratomas. If cells are transformed or tumorigenic, and it is desirable to use them for infusion, such cells may be disabled so they cannot form tumors *in vivo,* as by treatment that prevents cell proliferation into tumors. Such treatments are well known in the art.

In one embodiment, the subject is human.

The cells that express and/or secrete the modulatory factors can be used in drug discovery methods to screen for an agent that affects the ability of the cells to modulate the activation of macrophages as set out in the claims so as to be able achieve any of the above effects. Such agents include, but are not limited to, small organic molecules, antisense nucleic acids, siRNA, DNA aptamers, peptides, antibodies, non-antibody proteins, cytokines, chemokines, and chemo-attractants.

Because the effects described in this application can be caused by secreted factors, not only the cells, but also conditioned medium (and extracts thereof) produced from culturing the cells, is useful to achieve the effects. Such medium would contain the secreted factor(s) and, therefore, could be used instead of the cells or added to the cells. So, where cells can be used, it should be understood that conditioned medium (and extracts thereof) would also be effective and could be substituted or added.

In view of the property of the cells to achieve the above effects, cell banks can be established containing cells that are selected for having a desired potency to modulate the activation of macrophages as set out in the claims so as to be able to achieve any of the above effects. Accordingly, the invention encompasses assaying cells for the ability to modulate the activation of macrophages and banking the cells having a desired potency. The bank can provide a source for making a pharmaceutical composition to administer to a subject. Cells can be used directly from the bank or expanded prior to use. Especially in the case that the cells are subjected to further expansion, after expansion it is desirable to validate that the cells still have the desired potency. Banks allow "off the shelf" use of cells that are allogeneic to the subject.

Accordingly, the invention also is directed to diagnostic procedures conducted prior to administering the cells to a subject. The pre-diagnostic procedures include assessing the potency of the cells to modulate the activation of macrophages as set out in the claims so as to be able to achieve one or more of the above effects. The cells may be taken from a cell bank and used directly or expanded prior to administration. In either case, the cells could be assessed for the desired potency. Especially in the case that the cells are subjected to further expansion, after expansion it is desirable to validate that the cells still have the desired potency. Or the cells can be derived from the subject and expanded prior to administration. In this case, as well, the cells could be assessed for the desired potency prior to administration back to the subject (autologous).

Although the cells selected for modulation are necessarily assayed during the selection procedure, it may be preferable and prudent to again assay the cells prior to administration to a subject for treatment to confirm that the cells still modulate macrophage activation at desired levels. This is particularly preferable where the cells have been expanded or have been stored for any length of time, such as in a cell bank, where cells are most likely frozen during storage.

With respect to methods with cells that modulate the activation of macrophages, between the original isolation of the cells and the administration to a subject, there may be multiple (i.e., sequential) assays for modulation. This is to confirm that the cells can still modulate the activation of macrophages, at desired levels, after manipulations that occur within this time frame. For example, an assay may be performed after each expansion of the cells. If cells are stored in a cell bank, they may be assayed after being released from storage. If they are frozen, they may be assayed after thawing. If the cells from a cell bank are expanded, they may be assayed after expansion. Preferably, a portion of the final cell product (i.e., the cell preparation that is physically administered to the subject) may be assayed.

The invention is also directed to a method for establishing the dosage of the cells by assessing the potency of the cells to modulate the activation of macrophages so as to be able to achieve one or more of the above effects. In this case, the potency would be determined and the dosage adjusted accordingly.

Potency can be assessed by measuring the degree of macrophage activation *in vitro* by means of macrophage gene expression or by the effects of macrophage activation.

One may monitor macrophage function (e.g., activation and presentation) to establish and maintain a proper dosage regimen. One could monitor the function at various levels, such as *in vivo* by means of circulating secreted factors expressed by activated macrophages. One might also assay macrophages that are derived from the patient in *in vitro* assays of gene expression or macrophage function. Thus, the invention is directed to evaluating the dosage efficacy of the cells as an immunomodulator in the CNS in a patient by assessing and/or monitoring the *in vivo* activation of macrophage.

The methods and compositions of the invention are useful for treating any disease involving inflammation. This includes, but is not limited to, acute and chronic conditions in cardiovascular, e.g., acute myocardial infarction; peripheral vascular disease; pulmonary, e.g., asthma, ARDS; autoimmune, e.g., rheumatoid arthritis, multiple sclerosis, lupus, sclerodoma; psoriasis; gastrointestinal, e.g., graft-versus-host-disease, Crohn's disease, diabetes, ulcerative colitis, acute and chronic transplantation rejection, and dermatitis.

The methods and compositions of the invention are useful for treating any CNS condition involving inflammation, including, but not limited to, ischemic stroke, multiple sclerosis, Alzheimer's Disease, ALS, Parkinson's Disease, hypoxic-ischemia, neonatal hypoxic ischemia, and traumatic brain or spinal cord injury.

In one particular embodiment, the methods and compositions of the invention are used to treat traumatic brain injury or hypoxic ischemia.

For these treatments, one would administer the cells that modulate the activation of macrophages. Such cells could have been assessed for the capacity to modulate the activation of macrophages and selected for a desired degree of modulation.

It is understood, however, that for treatment of any of the above conditions, it may be expedient to use such cells; that is, one that has been assessed for modulating the activation of macrophages and selected for a desired level of modulation prior to administration for treatment of the condition.

In a highly specific embodiment, the pathology is traumatic brain injury or spinal cord injury and the cells are non-embryonic stem, non-germ cells that have extended replicative capacity and pluripotentiality and, therefore, express certain markers, e.g., one or more of telomerase, oct4, rex-1, and rox-1, and have broad differentiation potential, e.g., at least two of ectodermal, endodermal, and mesodermal cell types.

The inventors have also found, in a hypoxic ischemic injury model, that the numbers of macrophages normally found at the site of ischemic injury were severely reduced in number when cells described herein were administered intravenously to the ischemic subject (Example 2 in this application). The invention, therefore, is also broadly directed to using the cells described herein to reduce the infiltration of macrophages to the site of injury. Accordingly, treatment with the cells described herein may prevent macrophages from exiting the site at which they are normally activated, such as the spleen. In this case, in a clinical setting, one may administer the cells after obtaining a baseline by assaying for the presence of macrophages in the circulation, either directly or by means of a gene normally expressed by the macrophages and, then, following administration of the cells during treatment, monitor one or more times for the presence of the macrophages in the circulation. One could then determine the optimized dose for treatment that will result in the macrophage not entering the circulation to reach the site of injury.

### BRIEF DESCRIPTION OF THE FIGURES

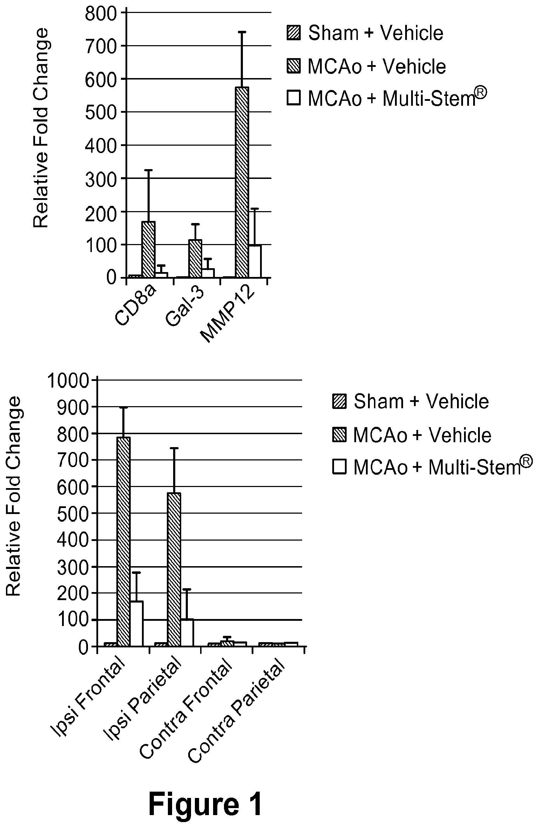

Figure 1 - MultiStem® significantly inhibits ischemia-induced markers of immune response within infarct region. qPCR analysis confirms that MultiStem® administration prevents massive ischemia-induced upregulation of markers of immune response, such as MMP12 (expressed by macrophages). qPCR analysis of different brain regions demonstrates that the changes in immune marker genes are specific to the infarct region.

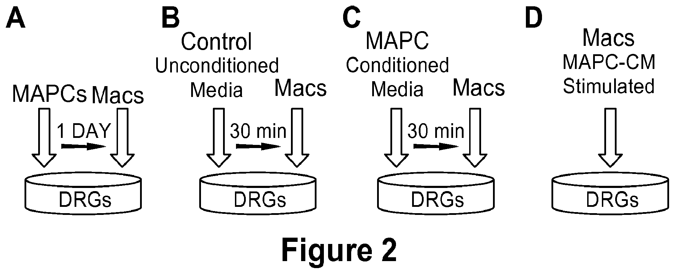

Figure 2 - Schematic summary of *in vitro* experiments. **(A)** MAPC added to DRG neuron culture 1 day prior to time-lapse imaging and addition of macrophages. **(B)** Control unconditioned media added to DRG neuron culture during time-lapse imaging, 30 minutes prior to macrophage addition. (C) MAPC-conditioned media added to DRG neuron culture during time-lapse imaging, 30 minutes prior to macrophage addition. **(D)** Macrophages that have been pretreated with MAPC-conditioned media are added to DRG neuron culture during time-lapse imaging.

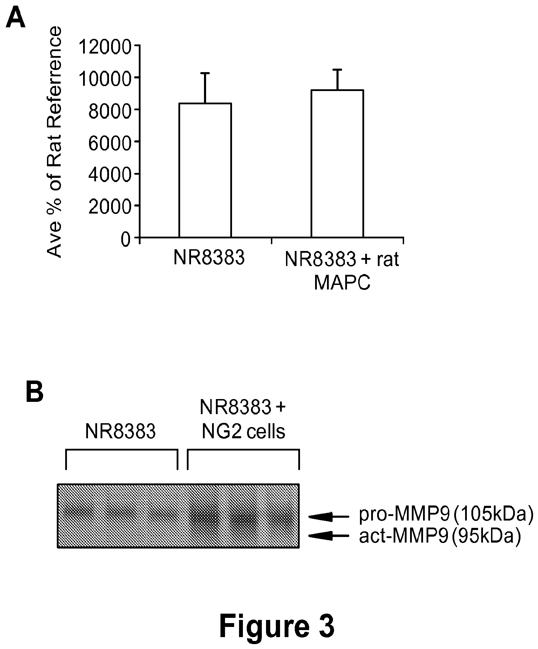

Figure 3 - **(A)** qPCR demonstrating MMP-9 transcript levels from macrophages in the presence or absence of MAPC. **(B)** Western blot of MMP-9 levels in NR8383 macrophages in the presence or absence of spinal cord-derived NG2+ cells. Background subtracted band densitometry was measured and analyzed by Student's t-test for statistical significance. Error bars indicate SEM.

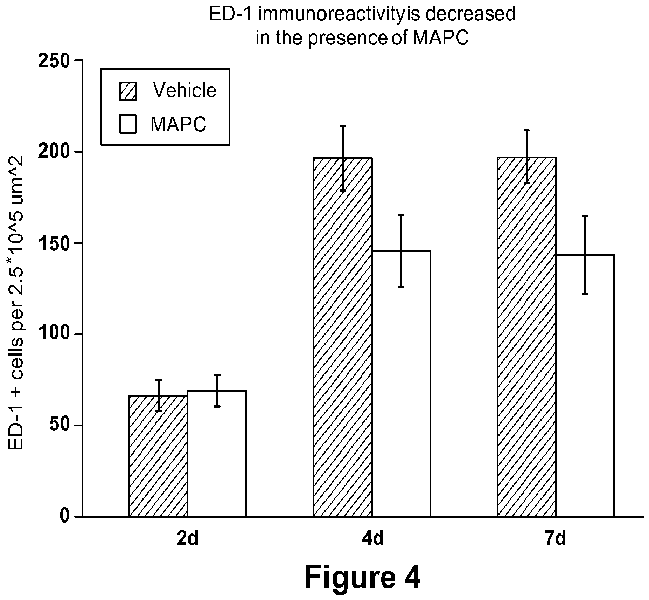

Figure 4 - Graphical representation of ED-1+ immunoreactivity in the center of the dorsal column crush lesion at 2, 4, and 7 days post injury in animals receiving Vehicle only or MAPC transplants. The conditions, Vehicle control and MAPC treated groups are significantly different from one another, p<0.01(two-way ANOVA, *F*(1,66)=7.05). Error bars indicate SEM.

### DETAILED DESCRIPTION OF THE INVENTION

It should be understood that this invention is not limited to the particular methodology, protocols, and reagents, etc., described herein and, as such, may vary. The terminology used herein is for the purpose of describing particular embodiments only, and is not intended to limit the scope of the disclosed invention, which is defined solely by the claims.

The section headings are used herein for organizational purposes only and are not to be construed as in any way limiting the subject matter described.

The methods and techniques of the present application are generally performed according to conventional methods well-known in the art and as described in various general and more specific references that are cited and discussed throughout the present specification unless otherwise indicated. See, e.g., Sambrook et al., Molecular Cloning: A Laboratory Manual, 3rd ed., Cold Spring Harbor Laboratory Press, Cold Spring Harbor, N.Y. (2001) and Ausubel et al., Current Protocols in Molecular Biology, Greene Publishing Associates (1992), and Harlow and Lane, Antibodies: A Laboratory Manual, Cold Spring Harbor Laboratory Press, Cold Spring Harbor, N.Y. (1990).

### Definitions

"A" or "an" means herein one or more than one; at least one. Where the plural form is used herein, it generally includes the singular.

A "cell bank" is industry nomenclature for cells that have been grown and stored for future use. Cells may be stored in aliquots. They can be used directly out of storage or may be expanded after storage. This is a convenience so that there are "off the shelf" cells available for administration. The cells may already be stored in a pharmaceutically-acceptable excipient so they may be directly administered or they may be mixed with an appropriate excipient when they are released from storage. Cells may be frozen or otherwise stored in a form to preserve viability. In one embodiment of the invention, cell banks are created in which the cells have been selected for enhanced modulation of activation of macrophages. Following release from storage, and prior to administration to the subject, it may be preferable to again assay the cells for potency, i.e., level of modulation of activation of macrophages. This can be done using any of the assays, direct or indirect, described in this application or otherwise known in the art. Then cells having the desired potency can then be administered to the subject for treatment. Banks can be made using cells derived from the individual to be treated (from their pre-natal tissues such as placenta, umbilical cord blood, or umbilical cord matrix or expanded from the individual at any time after birth) (autologous). Or banks can contain cells for allogeneic uses.

"Co-administer" means to administer in conjunction with one another, together, coordinately, including simultaneous or sequential administration of two or more agents.

"Comprising" means, without other limitation, including the referent, necessarily, without any qualification or exclusion on what else may be included. For example, "a composition comprising x and y" encompasses any composition that contains x and y, no matter what other components may be present in the composition. Likewise, "a method comprising the step of x" encompasses any method in which x is carried out, whether x is the only step in the method or it is only one of the steps, no matter how many other steps there may be and no matter how simple or complex x is in comparison to them. "Comprised of and similar phrases using words of the root "comprise" are used herein as synonyms of "comprising" and have the same meaning.

"Comprised of" is a synonym of "comprising" (see above).

"Conditioned cell culture medium" is a term well-known in the art and refers to medium in which cells have been grown. Herein this means that the cells are grown for a sufficient time to secrete the factors that are effective to achieve any of the results described in this application, including modulation of activation of macrophages.

Conditioned cell culture medium refers to medium in which cells have been cultured so as to secrete factors into the medium. For the purposes of the present invention, cells can be grown through a sufficient number of cell divisions so as to produce effective amounts of such factors so that the medium has the effects, including modulation of activation of macrophages, etc. Cells are removed from the medium by any of the known methods in the art, including, but not limited to, centrifugation, filtration, immunodepletion (e.g., via tagged antibodies and magnetic columns), and FACS sorting.

"EC cells" were discovered from analysis of a type of cancer called a teratocarcinoma. In 1964, researchers noted that a single cell in teratocarcinomas could be isolated and remain undifferentiated in culture. This type of stem cell became known as an embryonic carcinoma cell (EC cell).

"Effective amount" generally means an amount which provides the desired local or systemic effect, e.g., effective to ameliorate undesirable effects of inflammation, including modulation of activation of macrophages, etc. For example, an effective amount is an amount sufficient to effectuate a beneficial or desired clinical result. The effective amounts can be provided all at once in a single administration or in fractional amounts that provide the effective amount in several administrations. The precise determination of what would be considered an effective amount may be based on factors individual to each subject, including their size, age, injury, and/or disease or injury being treated, and amount of time since the injury occurred or the disease began. One skilled in the art will be able to determine the effective amount for a given subject based on these considerations which are routine in the art. As used herein, "effective dose" means the same as "effective amount."

"Effective route" generally means a route which provides for delivery of an agent to a desired compartment, system, or location. For example, an effective route is one through which an agent can be administered to provide at the desired site of action an amount of the agent sufficient to effectuate a beneficial or desired clinical result.

"Embryonic Stem Cells (ESC)" are well known in the art and have been prepared from many different mammalian species. Embryonic stem cells are stem cells derived from the inner cell mass of an early stage embryo known as a blastocyst. They are able to differentiate into all derivatives of the three primary germ layers: ectoderm, endoderm, and mesoderm. These include each of the more than 220 cell types in the adult body. The ES cells can become any tissue in the body, excluding placenta. Only the morula's cells are totipotent, able to become all tissues and a placenta. Some cells similar to ESCs may be produced by nuclear transfer of a somatic cell nucleus into an enucleated fertilized egg.

Use of the term "includes" is not intended to be limiting.

"Increase" or "increasing" with respect to increasing the neuroprotective activation state means to induce it entirely where there was no pre-existing activation or to increase the degree of neuroprotective activation.

"Induced pluripotent stem cells (IPSC or IPS cells)" are somatic cells that have been reprogrammed, for example, by introducing exogenous genes that confer on the somatic cell a less differentiated phenotype. These cells can then be induced to differentiate into less differentiated progeny. IPS cells have been derived using modifications of an approach originally discovered in 2006 (Yamanaka, S. et al., Cell Stem Cell, 1:39-49 (2007)). For example, in one instance, to create IPS cells, scientists started with skin cells that were then modified by a standard laboratory technique using retroviruses to insert genes into the cellular DNA. In one instance, the inserted genes were Oct4, Sox2, Lif4, and c-myc, known to act together as natural regulators to keep cells in an embryonic stem cell-like state. These cells have been described in the literature. See, for example, Wernig et al., PNAS, 105:5856-5861 (2008); Jaenisch et al., Cell, 132:567-582 (2008); Hanna et al., Cell, 133:250-264 (2008); and Brambrink et al., Cell Stem Cell, 2:151-159 (2008). These references are teaching IPSCs and methods for producing them. It is also possible that such cells can be created by specific culture conditions (exposure to specific agents).

The term "isolated" refers to a cell or cells which are not associated with one or more cells or one or more cellular components that are associated with the cell or cells *in vivo.* An "enriched population" means a relative increase in numbers of a desired cell relative to one or more other cell types *in vivo* or in primary culture.

However, as used herein, the term "isolated" does not indicate the presence of only stem cells. Rather, the term "isolated" indicates that the cells are removed from their natural tissue environment and are present at a higher concentration as compared to the normal tissue environment. Accordingly, an "isolated" cell population may further include cell types in addition to stem cells and may include additional tissue components. This also can be expressed in terms of cell doublings, for example. A cell may have undergone 10, 20, 30, 40 or more doublings *in vitro* or *ex vivo* so that it is enriched compared to its original numbers *in vivo* or in its original tissue environment (e.g., bone marrow, peripheral blood, adipose tissue, etc.).

"MAPC" is an acronym for "multipotent adult progenitor cell." It refers to a cell that is not an embryonic stem cell or germ cell but has some characteristics of these. MAPC can be characterized in a number of alternative descriptions, each of which conferred novelty to the cells when they were discovered. They can, therefore, be characterized by one or more of those descriptions. First, they have extended replicative capacity in culture without being transformed (tumorigenic) and with a normal karyotype. Second, they may give rise to cell progeny of more than one germ layer, such as two or all three germ layers (i.e., endoderm, mesoderm and ectoderm) upon differentiation. Third, although they are not embryonic stem cells or germ cells, they may express markers of these primitive cell types so that MAPCs may express one or more of Oct 3/4 (i.e., Oct 3A), rex-1, and rox-1. They may also express one or more of sox-2 and SSEA-4. Fourth, like a stem cell, they may self-renew, that is, have an extended replication capacity without being transformed. This means that these cells express telomerase (i.e., have telomerase activity). Accordingly, the cell type that was designated "MAPC" may be characterized by alternative basic characteristics that describe the cell via some of its novel properties.

The term "adult" in MAPC is non-restrictive. It refers to a non-embryonic somatic cell. MAPCs are karyotypically normal and do not form teratomas *in vivo.* This acronym was first used in U.S. Patent No. 7,015,037 to describe a pluripotent cell isolated from bone marrow. However, cells with pluripotential markers and/or differentiation potential have been discovered subsequently and, for purposes of this invention, may be equivalent to those cells first designated "MAPC." Essential descriptions of the MAPC type of cell are provided in the Summary of the Invention above.

MAPC represents a more primitive progenitor cell population than MSC (Verfaillie, C.M., Trends Cell Biol 12:502-8 (2002), Jahagirdar, B.N., et al., Exp Hematol, 29:543-56 (2001); Reyes, M. and C.M. Verfaillie, Ann N Y Acad Sci, 938:231-233 (2001); Jiang, Y. et al., Exp Hematol, 30896-904 (2002); and (Jiang, Y. et al., Nature, 418:41-9. (2002)).

The term "MultiStem®" is the trade name for a cell preparation based on the MAPCs of U.S. Patent No. 7,015,037, i.e., a non-embryonic stem, non-germ cell as described above. MultiStem® is prepared according to cell culture methods disclosed in this patent application, particularly, lower oxygen and higher serum.

This application refers to modulating macrophage activation. With respect to the invention, the modulatory cells cause a decrease in the macrophage activation state M1 and/or increase in M2 macrophages. Thus, the M1:M2 ratio decreases. This can be indicated, as disclosed herein, by changes in the levels of factors that are associated with M1 and M2 macrophages.

"Pharmaceutically-acceptable carrier" is any pharmaceutically-acceptable medium for the cells used in the present invention. Such a medium may retain isotonicity, cell metabolism, pH, and the like. It is compatible with administration to a subject *in vivo,* and can be used, therefore, for cell delivery and treatment.

The term "potency" refers to the ability of the cells (or conditioned medium from the cells) to achieve the various effects described in this application. Accordingly, potency refers to the effect at various levels, including, but not limited to, reducing symptoms of inflammation, modulating macrophage activation, etc.

"Primordial embryonic germ cells" (PG or EG cells) can be cultured and stimulated to produce many less differentiated cell types.

"Progenitor cells" are cells produced during differentiation of a stem cell that have some, but not all, of the characteristics of their terminally-differentiated progeny. Defined progenitor cells, such as "cardiac progenitor cells," are committed to a lineage, but not to a specific or terminally differentiated cell type. The term "progenitor" as used in the acronym "MAPC" does not limit these cells to a particular lineage. A progenitor cell can form a progeny cell that is more highly differentiated than the progenitor cell.

The term "reduce" as used herein means to prevent as well as decrease. In the context of treatment, to "reduce" is to either prevent or ameliorate one or more clinical symptoms. A clinical symptom is one (or more) that has or will have, if left untreated, a negative impact on the quality of life (health) of the subject. This also applies to the underlying biological effects such as reducing pro-inflammatory molecules, activation of macrophages, etc., the end result of which would be to ameliorate the deleterious effects of inflammation.

"Selecting" a cell with a desired level of potency (e.g., for modulating macrophage activation) can mean identifying (as by assay), isolating, and expanding a cell. This could create a population that has a higher potency than the parent cell population from which the cell was isolated. The "parent" cell population refers to the parent cells from which the selected cells are divided. "Parent" refers to an actual P1 → F1 relationship (i.e., a cell progeny). So if cell X is isolated from a mixed population of cells X and Y, in which X is an expressor and Y is not, one would not classify a mere isolate of X as having enhanced expression. But, if a progeny cell of X is a higher expressor, one would classify the progeny cell as having enhanced expression.

To select a cell that modulates macrophage activation would include both an assay to determine if there is modulation and would also include obtaining that cell. The cell may naturally modulate macrophage activation in that the cell was not incubated with or exposed to an agent that induces modulation of activation. The cell may not be known to be a modulator of macrophage activation prior to conducting the assay. As modulation could depend on gene expression and/or secretion, one could also select on the basis of one or more of the genes that cause modulation.

Selection could be from cells in a tissue. For example, in this case, cells would be isolated from a desired tissue, expanded in culture, selected for modulation of activation of macrophages, and the selected cells further expanded.

Selection could also be from cells *ex vivo,* such as cells in culture. In this case, one or more of the cells in culture would be assayed for modulation of activation of macrophages and the cells obtained that modulate the activation of macrophages could be further expanded.

Cells could also be selected for enhanced modulation of activation of macrophages. In this case, the cell population from which the enhanced cell is obtained already modulates the activation of macrophages. Enhanced modulation of activation of macrophages means a higher average amount (activation of macrophages) per cell than in the parent population.

The parent population from which the enhanced cell is selected may be substantially homogeneous (the same cell type). One way to obtain such an enhanced cell from this population is to create single cells or cell pools and assay those cells or cell pools for modulation of activation of macrophages to obtain clones that naturally modulate the activation of macrophages (as opposed to treating the cells with a modulation of activation of macrophages inducer) and then expanding those cells that are naturally enhanced.

However, cells may be treated with one or more agents that will enhance modulation of activation of macrophages of the endogenous cellular pathways. Thus, substantially homogeneous populations may be treated to enhance modulation.

If the population is not substantially homogeneous, then, it is preferable that the parental cell population to be treated contains at least 100 of the modulator cell type in which enhanced modulation is sought, more preferably at least 1,000 of the cells, and still more preferably, at least 10,000 of the cells. Following treatment, this sub-population can be recovered from the heterogeneous population by known cell selection techniques and further expanded if desired.

Thus, desired levels of modulation of activation of macrophages may be those that are higher than the levels in a given preceding population. For example, cells that are put into primary culture from a tissue and expanded and isolated by culture conditions that are not specifically designed to promote modulation of activation of macrophages, may provide a parent population. Such a parent population can be treated to enhance the average modulation of activation of macrophages per cell or screened for a cell or cells within the population that express higher modulation of activation of macrophages without deliberate treatment. Such cells can be expanded then to provide a population with a higher (desired) expression.

"Self-renewal" refers to the ability to produce replicate daughter stem cells having differentiation potential that is identical to those from which they arose. A similar term used in this context is "proliferation."

"Stem cell" means a cell that can undergo self-renewal (i.e., progeny with the same differentiation potential) and also produce progeny cells that are more restricted in differentiation potential. Within the context of the invention, a stem cell would also encompass a more differentiated cell that has de-differentiated, for example, by nuclear transfer, by fusion with a more primitive stem cell, by introduction of specific transcription factors, or by culture under specific conditions. See, for example, Wilmut et al., Nature, 385:810-813 (1997); Ying et al., Nature, 416:545-548 (2002); Guan et al., Nature, 440:1199-1203 (2006); Takahashi et al., Cell, 126:663-676 (2006); Okita et al., Nature, 448:313-317 (2007); and Takahashi et al., Cell, 131:861-872 (2007).

Dedifferentiation may also be caused by the administration of certain compounds or exposure to a physical environment *in vitro* or *in vivo* that would cause the dedifferentiation. Stem cells also may be derived from abnormal tissue, such as a teratocarcinoma. Stem cells may also be produced by introducing genes associated with stem cell function into a non-stem cell, such as an induced pluripotent stem cell.

"Subject" means a vertebrate, such as a mammal, such as a human. Mammals include, but are not limited to, humans, dogs, cats, horses, cows, and pigs.

The term "therapeutically effective amount" refers to the amount of an agent determined to produce any therapeutic response in a mammal. For example, effective anti-inflammatory therapeutic agents may prolong the survivability of the patient, and/or inhibit overt clinical symptoms. Treatments that are therapeutically effective within the meaning of the term as used herein, include treatments that improve a subject's quality of life even if they do not improve the disease outcome per se. Such therapeutically effective amounts are readily ascertained by one of ordinary skill in the art. Thus, to "treat" means to deliver such an amount. Thus, treating can prevent or ameliorate any pathological symptoms of inflammation.

"Treat," "treating," or "treatment" are used broadly in relation to the invention and each such term encompasses, among others, preventing, ameliorating, inhibiting, or curing a deficiency, dysfunction, disease, or other deleterious process, including those that interfere with and/or result from a therapy. Treating an undesirable inflammatory component, within the context of the invention, refers to inflammation that includes clinically detrimental macrophage activation as a component.

"Validate" means to confirm. In the context of the invention, one confirms that a cell is an expressor with a desired potency. This is so that one can then use that cell (in treatment, banking, drug screening, etc.) with a reasonable expectation of efficacy. Accordingly, to validate means to confirm that the cells, having been originally found to have/established as having modulatory activity, in fact, retain that activity. Thus, validation is a verification event in a two-event process involving the original determination and the follow-up determination. The second event is referred to herein as "validation."

### Stem Cells

The present invention can be practiced, preferably, using stem cells of vertebrate species, such as humans, non-human primates, domestic animals, livestock, and other non-human mammals.

The claims are limited to the use of MAPCs. MAPC

Human MAPCs are described in U.S. Patent 7,015,037. MAPCs have been identified in other mammals. Murine MAPCs, for example, are also described in U.S. Patent 7,015,037. Rat MAPCs are also described in U.S. Patent No. 7,838,289.

These references are describing MAPCs first isolated by Catherine Verfaillie.

### Isolation and Growth of MAPCs

Methods of MAPC isolation are known in the art. See, for example, U.S. Patent 7,015,037, along with the characterization (phenotype) of MAPCs.

MAPCs can be isolated from multiple sources, including, but not limited to, bone marrow, placenta, umbilical cord and cord blood, muscle, brain, liver, spinal cord, blood or skin. It is, therefore, possible to obtain bone marrow aspirates, brain or liver biopsies, and other organs, and isolate the cells using positive or negative selection techniques available to those of skill in the art, relying upon the genes that are expressed (or not expressed) in these cells (e.g., by functional or morphological assays such as those disclosed in the above-referenced applications)

MAPCs have also been obtained my modified methods described in Breyer et al., Experimental Hematology, 34:1596-1601 (2006) and Subramanian et al., Cellular Programming and Reprogramming: Methods and Protocols; S. Ding (ed.), Methods in Molecular Biology, 636:55-78 (2010). MAPCs from Human Bone Marrow as Described in U.S. Patent 7,015,037

MAPCs do not express the common leukocyte antigen CD45 or erythroblast specific glycophorin-A (Gly-A). The mixed population of cells was subjected to a Ficoll Hypaque separation. The cells were then subjected to negative selection using anti-CD45 and anti-Gly-A antibodies, depleting the population of CD45⁺ and Gly-A⁺ cells, and the remaining approximately 0.1% of marrow mononuclear cells were then recovered. Cells could also be plated in fibronectin-coated wells and cultured as described below for 2-4 weeks to deplete the cells of CD45⁺ and Gly-A⁺ cells. In cultures of adherent bone marrow cells, many adherent stromal cells undergo replicative senescence around cell doubling 30 and a more homogenous population of cells continues to expand and maintains long telomeres.

Alternatively, positive selection could be used to isolate cells via a combination of cell-specific markers. Both positive and negative selection techniques are available to those of skill in the art, and numerous monoclonal and polyclonal antibodies suitable for negative selection purposes are also available in the art (see, for example, Leukocyte Typing V, Schlossman, et al., Eds. (1995) Oxford University Press) and are commercially available from a number of sources.

Techniques for mammalian cell separation from a mixture of cell populations have also been described by Schwartz, et al., in U. S. Patent No. 5,759,793 (magnetic separation), Basch et al., 1983 (immunoaffinity chromatography), and Wysocki and Sato, 1978 (fluorescence-activated cell sorting).

Cells may be cultured in low-serum or serum-free culture medium. Serum-free medium used to culture MAPCs is described in U.S. Patent 7,015,037. Commonly-used growth factors include but are not limited to platelet-derived growth factor and epidermal growth factor. See, for example, U.S. Patent Nos. 7,169,610; 7,109,032; 7,037,721; 6,617,161; 6,617,159; 6,372,210;6,224,860; 6,037,174; 5,908,782; 5,766,951; 5,397,706; and 4,657,866.

### Additional Culture Methods

In additional experiments the density at which MAPCs are cultured can vary from about 100 cells/cm² or about 150 cells/cm² to about 10,000 cells/cm², including about 200 cells/cm² to about 1500 cells/cm² to about 2000 cells/cm². The density can vary between species. Additionally, optimal density can vary depending on culture conditions and source of cells. It is within the skill of the ordinary artisan to determine the optimal density for a given set of culture conditions and cells.

Also, effective atmospheric oxygen concentrations of less than about 10%, including about 1-5% and, especially, 3-5%, can be used at any time during the isolation, growth and differentiation of MAPCs in culture.

Cells may be cultured under various serum concentrations, e.g., about 2-20%. Fetal bovine serum may be used. Higher serum may be used in combination with lower oxygen tensions, for example, about 15-20%. Cells need not be selected prior to adherence to culture dishes. For example, after a Ficoll gradient, cells can be directly plated, e.g., 250,000-500,000/cm². Adherent colonies can be picked, possibly pooled, and expanded.

In one embodiment, used in the experimental procedures in the Examples, high serum (around 15-20%) and low oxygen (around 3-5%) conditions were used for the cell culture. Specifically, adherent cells from colonies were plated and passaged at densities of about 1700-2300 cells/cm² in 18% serum and 3% oxygen (with PDGF and EGF).

In an embodiment specific for MAPCs, supplements are cellular factors or components that allow MAPCs to retain the ability to differentiate into cell types of more than one embryonic lineage, such as all three lineages. This may be indicated by the expression of specific markers of the undifferentiated state, such as Oct 3/4 (Oct 3A) and/or markers of high expansion capacity, such as telomerase.

### Cell Culture

For all the components listed below, see U.S. 7,015,037.

In general, cells useful for the invention can be maintained and expanded in culture medium that is available and well-known in the art. Also contemplated is supplementation of cell culture medium with mammalian sera. Additional supplements can also be used advantageously to supply the cells with the necessary trace elements for optimal growth and expansion. Hormones can also be advantageously used in cell culture. Lipids and lipid carriers can also be used to supplement cell culture media, depending on the type of cell and the fate of the differentiated cell. Also contemplated is the use of feeder cell layers.

Cells in culture can be maintained either in suspension or attached to a solid support, such as extracellular matrix components. Stem cells often require additional factors that encourage their attachment to a solid support, such as type I and type II collagen, chondroitin sulfate, fibronectin, "superfibronectin" and fibronectin-like polymers, gelatin, poly-D and poly-L-lysine, thrombospondin and vitronectin. One embodiment of the present invention utilizes fibronectin. See, for example, Ohashi et al., Nature Medicine, 13:880-885 (2007); Matsumoto et al., J Bioscience and Bioengineering, 105:350-354 (2008); Kirouac et al., Cell Stem Cell, 3:369-381 (2008); Chua et al., Biomaterials, 26:2537-2547 (2005); Drobinskaya et al., Stem Cells, 26:2245-2256 (2008); Dvir-Ginzberg et al., FASEB J, 22:1440-1449 (2008); Turner et al., J Biomed Mater Res Part B: Appl Biomater, 82B:156-168 (2007); and Miyazawa et al., Journal of Gastroenterology and Hepatology, 22:1959-1964 (2007)).

Cells may also be grown in "3D" (aggregated) cultures. An example is PCT/US2009/31528, filed January 21, 2009.

Once established in culture, cells can be used fresh or frozen and stored as frozen stocks, using, for example, DMEM with 40% FCS and 10% DMSO. Other methods for preparing frozen stocks for cultured cells are also available to those of skill in the art.

### Pharmaceutical Formulations

U.S. 7,015,037 discloses pharmaceutical formulations. In certain embodiments, the cell populations are present within a composition adapted for and suitable for delivery, i.e., physiologically compatible.

In some embodiments the purity of the cells (or conditioned medium) for administration to a subject is about 100% (substantially homogeneous). In other embodiments it is 95% to 100%. In some embodiments it is 85% to 95%. Particularly, in the case of admixtures with other cells, the percentage can be about 10%-15%, 15%-20%, 20%-25%, 25%-30%, 30%-35%, 35%-40%, 40%-45%, 45%-50%, 60%-70%, 70%-80%, 80%-90%, or 90%-95%. Or isolation/purity can be expressed in terms of cell doublings where the cells have undergone, for example, 10-20, 20-30, 30-40, 40-50 or more cell doublings.

The choice of formulation for administering the cells for a given application will depend on a variety of factors. Prominent among these will be the species of subject, the nature of the condition being treated, its state and distribution in the subject, the nature of other therapies and agents that are being administered, the optimum route for administration, survivability via the route, the dosing regimen, and other factors that will be apparent to those skilled in the art. For instance, the choice of suitable carriers and other additives will depend on the exact route of administration and the nature of the particular dosage form.

Final formulations of the aqueous suspension of cells/medium will typically involve adjusting the ionic strength of the suspension to isotonicity (i.e., about 0.1 to 0.2) and to physiological pH (i.e., about pH 6.8 to 7.5). The final formulation will also typically contain a fluid lubricant.

In some embodiments, cells/medium are formulated in a unit dosage injectable form, such as a solution, suspension, or emulsion. Pharmaceutical formulations suitable for injection of cells/medium typically are sterile aqueous solutions and dispersions. Carriers for injectable formulations can be a solvent or dispersing medium containing, for example, water, saline, phosphate buffered saline, polyol (for example, glycerol, propylene glycol, liquid polyethylene glycol, and the like), and suitable mixtures thereof.

The skilled artisan can readily determine the amount of cells and optional additives, vehicles, and/or carrier in compositions to be administered in methods of the invention. Typically, any additives (in addition to the cells) are present in an amount of 0.001 to 50 wt % in solution, such as in phosphate buffered saline. The active ingredient is present in the order of micrograms to milligrams, such as about 0.0001 to about 5 wt %, preferably about 0.0001 to about 1 wt %, most preferably about 0.0001 to about 0.05 wt % or about 0.001 to about 20 wt %, preferably about 0.01 to about 10 wt %, and most preferably about 0.05 to about 5 wt %.

In some embodiments cells are encapsulated for administration, particularly where encapsulation enhances the effectiveness of the therapy, or provides advantages in handling and/or shelf life. Cells may be encapsulated by membranes, as well as capsules, prior to implantation. It is contemplated that any of the many methods of cell encapsulation available may be employed.

A wide variety of materials may be used in various embodiments for microencapsulation of cells. Such materials include, for example, polymer capsules, alginate-poly-L-lysine-alginate microcapsules, barium poly-L-lysine alginate capsules, barium alginate capsules, polyacrylonitrile/polyvinylchloride (PAN/PVC) hollow fibers, and polyethersulfone (PES) hollow fibers.

Techniques for microencapsulation of cells that may be used for administration of cells are known to those of skill in the art and are described, for example, in Chang, P., et al., 1999; Matthew, H.W., et al., 1991; Yanagi, K., et al., 1989; Cai Z.H., et al., 1988; Chang, T.M., 1992 and in U.S. Patent No. 5,639,275 (which, for example, describes a biocompatible capsule for long-term maintenance of cells that stably express biologically active molecules. Additional methods of encapsulation are in European Patent Publication No. 301,777 and U.S. Pat. Nos. 4,353,888; 4,744,933; 4,749,620; 4,814,274; 5,084,350; 5,089,272; 5,578,442; 5,639,275; and 5,676,943.

Certain embodiments incorporate cells into a polymer, such as a biopolymer or synthetic polymer. Examples of biopolymers include, but are not limited to, fibronectin, fibrin, fibrinogen, thrombin, collagen, and proteoglycans. Other factors, such as the cytokines discussed above, can also be incorporated into the polymer. In other embodiments of the invention, cells may be incorporated in the interstices of a three-dimensional gel. A large polymer or gel, typically, will be surgically implanted. A polymer or gel that can be formulated in small enough particles or fibers can be administered by other common, more convenient, non-surgical routes.

The dosage of the cells will vary within wide limits and will be fitted to the individual requirements in each particular case. In general, in the case of parenteral administration, it is customary to administer from about 0.01 to about 20 million cells/kg of recipient body weight. The number of cells will vary depending on the weight and condition of the recipient, the number or frequency of administrations, and other variables known to those of skill in the art. The cells can be administered by a route that is suitable for the tissue or organ. For example, they can be administered systemically, i.e., parenterally, by intravenous administration, or can be targeted to a particular tissue or organ; they can be administrated via subcutaneous administration or by administration into specific desired tissues.

The cells can be suspended in an appropriate excipient in a concentration from about 0.01 to about 5x10⁶ cells/ml. Suitable excipients for injection solutions are those that are biologically and physiologically compatible with the cells and with the recipient, such as buffered saline solution or other suitable excipients. The composition for administration can be formulated, produced, and stored according to standard methods complying with proper sterility and stability.

### Administration into Lymphohematopoietic Tissues

Techniques for administration into these tissues are known in the art. For example, intra-bone marrow injections can involve injecting cells directly into the bone marrow cavity typically of the posterior iliac crest but may include other sites in the iliac crest, femur, tibia, humerus, or ulna; splenic injections could involve radiographic guided injections into the spleen or surgical exposure of the spleen via laparoscopic or laparotomy; Peyer's patches, GALT, or BALT injections could require laparotomy or laparoscopic injection procedures.

### Dosing

Doses for humans or other mammals can be determined without undue experimentation by the skilled artisan, from this disclosure, the documents cited herein, and the knowledge in the art. The dose of cells/medium appropriate to be used in accordance with various embodiments of the invention will depend on numerous factors. The parameters that will determine optimal doses to be administered for primary and adjunctive therapy generally will include some or all of the following: the disease being treated and its stage; the species of the subject, their health, gender, age, weight, and metabolic rate; the subject's immunocompetence; other therapies being administered; and expected potential complications from the subject's history or genotype. The parameters may also include: whether the cells are syngeneic, autologous, allogeneic, or xenogeneic; their potency (specific activity); the site and/or distribution that must be targeted for the cells/medium to be effective; and such characteristics of the site such as accessibility to cells/medium and/or engraftment of cells. Additional parameters include co-administration with other factors (such as growth factors and cytokines). The optimal dose in a given situation also will take into consideration the way in which the cells/medium are formulated, the way they are administered, and the degree to which the cells/medium will be localized at the target sites following administration.

The optimal dose of cells could be in the range of doses used for autologous, mononuclear bone marrow transplantation. For fairly pure preparations of cells, optimal doses in various embodiments will range from 10⁴ to 10⁸ cells/kg of recipient mass per administration. In some embodiments the optimal dose per administration will be between 10⁵ to 10⁷ cells/kg. In many embodiments the optimal dose per administration will be 5 x 10⁵ to 5 x 10⁶ cells/kg. By way of reference, higher doses in the foregoing are analogous to the doses of nucleated cells used in autologous mononuclear bone marrow transplantation. Some of the lower doses are analogous to the number of CD34⁺ cells/kg used in autologous mononuclear bone marrow transplantation.

In various embodiments, cells/medium may be administered in an initial dose, and thereafter maintained by further administration. Cells/medium may be administered by one method initially, and thereafter administered by the same method or one or more different methods. The levels can be maintained by the ongoing administration of the cells/medium. Various embodiments administer the cells/medium either initially or to maintain their level in the subject or both by intravenous injection. In a variety of embodiments, other forms of administration are used, dependent upon the patient's condition and other factors, discussed elsewhere herein.

Cells/medium may be administered in many frequencies over a wide range of times. Generally lengths of treatment will be proportional to the length of the disease process, the effectiveness of the therapies being applied, and the condition and response of the subject being treated.

### Uses

Administering the cells is useful to reduce undesirable inflammation in any number of pathologies, including, but not limited to, colitis, alveolitis, bronchiolitis obliterans, ileitis, pancreatitis, glomerulonephritis, uveitis, arthritis, hepatitis, dermatitis, and enteritis, acute and chronic conditions in cardiovascular, e.g., acute myocardial infarction; central nervous system injury, e.g., stroke, traumatic brain injury, spinal cord injury; peripheral vascular disease; pulmonary, e.g., asthma, ARDS; autoimmune, e.g., rheumatoid arthritis, multiple sclerosis, lupus, sclerodoma; psoriasis; gastrointestinal, e.g., graft-versus-host-disease, Crohn's disease, diabetes, ulcerative colitis, acute and chronic transplantation rejection, dermatitis, colitis, alveolitis, bronchiolitis obliterans, ileitis, pancreatitis, glomerulonephritis, uveitis, arthritis, hepatitis, dermatitis, and enteritis.

Administering the cells is useful to reduce undesirable inflammation in any number of CNS pathologies, including, but not limited to, ischemic stroke, multiple sclerosis, Alzheimer's Disease, ALS, Parkinson's Disease, hypoxic-ischemia, neonatal hypoxic ischemia, and traumatic brain or spinal cord injury.

In addition, other uses are provided by knowledge of the biological mechanisms described in this application. One of these includes drug discovery. This aspect involves screening one or more compounds for the ability to affect the cell's ability to modulate the activation of macrophages. This would involve an assay for the cell's ability to modulate the activation of macrophages. Accordingly, the assay may be designed to be conducted *in vivo* or *in vitro.* Modulation assays could assess the activation state at any desired level, e.g., morphological, gene expression, functional, etc.

Cells (or medium) can be selected by the ability to modulate or by the expression of one or more genes that act as modulators. Gene expression can be assessed by directly assaying protein or RNA. This can be done through any of the well-known techniques available in the art, such as by FACS and other antibody-based detection methods and PCR and other hybridization-based detection methods. Indirect assays may also be used for expression, such as binding to any of the known receptors

Assays for potency may be performed by detecting the genes modulated by the cells. These may include, but are not limited to, oxygen radicals, NO, TNFα, Glu, quinolic acid, histamine, eicosanoids, NGF, BDNF, NT-4/5, TGFβ, GDNF, CNTF, IL-6, LIF, bFGF, HGF, PGn, IL-3, MMP-9, iNOS, CD16, CD86, CD64, and CD32, scavenger receptor A, CD163, arginase 1, CD14, CD206, CD23, and scavenger receptor B. Detection may be direct, e.g., via RNA or protein assays or indirect, e.g., biological assays for one or more biological effects of these genes. Alternatively, potency may be assayed by detecting the modulators of these genes, e.g., MMP modulators.

Accordingly, a surrogate marker could be used as long as it serves as an indicator that the cells express/secrete the factor(s) that modulate macrophage activation.

Assays for expression/secretion of modulatory factors include, but are not limited to, ELISA, Luminex. qRT-PCR, anti-factor western blots, and factor immunohistochemistry on tissue samples or cells.

Quantitative determination of modulatory factors in cells and conditioned media can be performed using commercially available assay kits (e.g., R&D Systems that relies on a two-step subtractive antibody-based assay).

*In vitro* activation assays can also be used to assess the expression/secretion of the factors. Such *in vitro* assays are well known in the art, for example, ELISA assay of secreted cytokines and growth factors, qRT-PCR analysis of genes associated with neurotoxic (M1) or neuroprotective (M2) activation state, and immunohistochemistry analysis of activation state marker expression.

A further use for the invention is the establishment of cell banks to provide cells for clinical administration. Generally, a fundamental part of this procedure is to provide cells that have a desired potency for administration in various therapeutic clinical settings.

Any of the same assays useful for drug discovery could also be applied to selecting cells for the bank as well as from the bank for administration.

Accordingly, in a banking procedure, the cells (or medium) would be assayed for the ability to achieve any of the above effects. Then, cells would be selected that have a desired potency for any of the above effects, and these cells would form the basis for creating a cell bank.

It is also contemplated that potency can be increased by treatment with an exogenous compound, such as a compound discovered through screening the cells with large combinatorial libraries. These compound libraries may be libraries of agents that include, but are not limited to, small organic molecules, antisense nucleic acids, siRNA DNA aptamers, peptides, antibodies, non-antibody proteins, cytokines, chemokines, and chemo-attractants. For example, cells may be exposed such agents at any time during the growth and manufacturing procedure. The only requirement is that there be sufficient numbers for the desired assay to be conducted to assess whether or not the agent increases potency. Such an agent, found during the general drug discovery process described above, could more advantageously be applied during the last passage prior to banking.

One embodiment that has been applied successfully to MultiStem is as follows. Cells can be isolated from a qualified marrow donor that has undergone specific testing requirements to determine that a cell product that is obtained from this donor would be safe to be used in a clinical setting. The mononuclear cells are isolated using either a manual or automated procedure. These mononuclear cells are placed in culture allowing the cells to adhere to the treated surface of a cell culture vessel. The MAPC cells are allowed to expand on the treated surface with media changes occurring on day 2 and day 4. On day 6, the cells are removed from the treated substrate by either mechanical or enzymatic means and replated onto another treated surface of a cell culture vessel. On days 8 and 10, the cells are removed from the treated surface as before and replated. On day 13, the cells are removed from the treated surface, washed and combined with a cryoprotectant material and frozen, ultimately, in liquid nitrogen. After the cells have been frozen for at least one week, an aliquot of the cells is removed and tested for potency, identity, sterility and other tests to determine the usefulness of the cell bank. These cells in this bank can then be used by thawing them, placing them in culture or use them out of the freeze to treat potential indications.

Another use is a diagnostic assay for efficiency and beneficial clinical effect following administration of the cells. Depending on the indication, there may be biomarkers available to assess. For example, one could assess circulating macrophages for specific markers of M1 and M2 macrophages. These are, for example, down-regulation of pro-inflammatory cytokines (TNF-α, 11-6, IL-1β, INF-γ) and up-regulation of anti-inflammatory cytokines (IL-4, IL-10, TGF-β). The dosage of cells can be adjusted during treatment according to the effect.

A further use is to assess the efficacy of the cell to achieve any of the above results as a pre-treatment diagnostic that precedes administering the cells to a subject. Moreover, dosage can depend upon the potency of the cells that are being administered. Accordingly, a pre-treatment diagnostic assay for potency can be useful to determine the dose of the cells initially administered to the patient and, possibly, further administered during treatment based on the real-time assessment of clinical effect.

It is also to be understood that the cells of the invention can be used to modulate activation not only for purposes of treatment, but also research purposes, both *in vivo* and *in vitro* to understand the mechanism involved in macrophage activation, normally and in disease models. In one embodiment, assays, *in vivo* or *in vitro,* can be done in the presence of desired agents. The effect of those agents can then be assessed. These types of assay could also be used to screen for agents that have an effect on the activation that is modulated by the cells of the invention. Accordingly, in one embodiment, one could screen for agents in the disease model that reverse the negative effects and/or promote positive effects. Conversely, one could screen for agents that have negative effects in a model of normal activation.

### Compositions

The invention is also directed to cell populations obtained with specific potencies for achieving any of the effects described herein. As described above, these populations are established by selecting for cells that have desired potency. These populations are used to make other compositions, for example, a cell bank comprising populations with specific desired potencies and pharmaceutical compositions containing a cell population with a specific desired potency.

### NON-LIMITING EXAMPLES

### Example 1

### Multipotent Adult Progenitor Cells (MAPC) Significantly Decrease Matrix Metalloproteinase-9 (MMP-9) Release From Macrophages

### Summary

Using an *in vitro* model of axonal dieback as well as a dorsal column crush model of spinal cord injury the inventors found that adult stem cells exert positive immunomodulatory and neurotrophic influences following acute injury to the nervous system. MAPC can affect both macrophages and dystrophic neurons simultaneously. They significantly decrease matrix metalloproteinase-9 (MMP-9) release from macrophages, effectively preventing induction of axonal dieback.

### Background

Previous work from the inventors has shown that the infiltration of phagocytic ED-1+ macrophages coincides with long distance axonal retraction, or dieback, from the initial site of a spinal cord lesion, resulting in a disadvantageous starting position for regenerating axons (Horn et al., J Neurosci 28:9330 (2008)). Injured axons that become dystrophic due to the presence of inhibitory proteoglycans in their environment are particularly susceptible to attack by macrophages. Several factors can affect the outcome of macrophage attack, including enhancement of the intrinsic growth potential of the neuron and alteration of macrophage-secreted factors (Busch et al., J Neurosci 29:9967 (2009)).

The inventors also previously have shown that macrophage-mediated axonal dieback can be prevented *in vitro* by specific inhibition of MMP-9. Additionally, endogenous stem-like NG2+ progenitor cells that normally populate the lesion center can stabilize axons undergoing axonal dieback as a result of their expression of growth promoting extracellular matrix molecules. But they cannot prevent the initiation of dieback (Busch et al., J Neurosci 30:255 (2010)).

In the past decade, adult stem cells have been isolated from multiple tissues and characterized in many laboratories (Mays et al., Expert Opin Biol Ther 7:173 (2007) and Ting et al., Crit Rev Oncol Hematol 65:81 (2008)). There is substantial interest in developing adult-derived stem cells as therapeutic agents for the treatment of CNS injury and disease (Biernaskie et al., J Neurosci 27:9545 (2007); Bambakidis et al., Neurosurg Focus 24:E20 (2008); and Barnabe-Heider et al., Cell Stem Cell 3:16 (2008)). Adult-derived adherent stem cells demonstrate a number of favorable characteristics including genetic stability, extensive expansion capacity, and low immunogenicity profiles that support allogeneic utility (Gnecchi et al., Circulation Research 103:1204 (2008)). Functional improvement after administration of adult-derived cells has been demonstrated in multiple pre-clinical models of injury or disease; however, many aspects of their mechanism of action remain poorly understood (Caplan et al., J Cell Biochem 98:1076 (2006); Kim et al., J Neurosci Res 87:2183 (2009); and Van't Hof et al., Cytotherapy 9:477 (2007)).

There is increasing evidence that transplanted adult stem cells tend to remain undifferentiated and often are cleared after a short time, but still promote recovery (Parr et al., Bone Marrow Transplant 40:609 (2007)). This suggests that their beneficial effects are mediated primarily through paracrine activities that impact host tissues. This has been attributed to mechanisms involving release of factors that are neuroprotective (Parr et al. 2007), angiogenic (Onda et al., J Cereb Blood Flow Metab 28:329 (2008)) and/or immunomodulatory (Ohtaki et al., Proc. Nat. Acad. Sci. U.S.A. 105:14638 (2008); Abrams et al., Restor Neurol Neurosci 27:307 (2009); and Boucherie et al., J Neurosci Res 87:1509 (2009)).

### Strategy

MAPC designates a well-characterized population of adherent stem cells isolated from adult bone marrow and other adult tissues (Jiang et al., Nature 418:41 (2002) and Jiang et al., Exp Hematol 30:896 (2002)) that can positively modulate the immune system (Kovacsovics-Bankowski et al., Cell Immunol 255:55 (2009) and Highfill et al., Blood 114:693 (2009)). Because of previous observations of the negative effects of macrophages after injury the inventors sought to determine if MAPC could affect immune cells responding to the initial insult to prevent axonal dieback and promote regeneration. The inventors tested the effects of MAPC on macrophage-mediated axonal dieback *in vitro* (Horn et al. 2008).

### Results

In the *in vitro* dieback model, in the presence of MAPC, NR8383 macrophages formed contacts with dystrophic axons that were often transient and rapidly broken. Five out of the six imaged axons in co-culture with MAPC did not undergo macrophage-mediated axonal retraction.

The ability of MAPC to prevent axonal dieback could be due to stimulatory effects on the neuron, modulatory effects on the macrophages, or a combination of both. To determine if MAPC-secreted factors were sufficient to modulate the retraction-inducing capabilities of macrophages, the inventors pretreated macrophages with medium conditioned by MAPC (MAPC-CM). The inventors observed no obvious differences in the ability of MAPC-CM-pretreated macrophages to recognize and tightly associate with dystrophic axons compared to untreated macrophages. However, macrophage-mediated axonal retraction still did not occur, indicating that MAPC-secreted factors were modulating the ability of the macrophages to induce dieback. Because the macrophages still formed long lasting contacts with the axon, it did not appear that their ability to recognize the dystrophic/injured state or adhere to the axon was impaired, but this activity alone was insufficient to induce dieback after pretreatment with MAPC.

Macrophages are known to secrete a variety of proteases, which aid in the breakdown and clearance of debris after injury (Yong, Nature reviews 6:931 (2005)). MMPs have been implicated in regeneration failure after CNS injury (Noble et al., J Neurosci 22:7526 (2002) and work from the inventors' laboratory has demonstrated that specific chemical inhibition of macrophage-produced MMP-9 can prevent axonal dieback (Busch et al. 2009). The inventors therefore examined the effect of MAPC on MMP-9 production and secretion from macrophages. The inventors found that co-culture with MAPC dramatically reduced the secretion of both the 105 kDa pro- and 95 kDa activated forms of MMP-9 by macrophages as measured by Western blot (P<0.05) and levels of functional protein as measured by gelatin zymography (P<0.05). Inhibition of MMP-9 proteolytic activity by binding of tissue inhibitor of metalloproteinases (TIMPs) is not a factor in the decreased MMP-9 levels observed by Western blot and zymogram, as the denaturing conditions present in these assays would disrupt any existing MMP-9 / TIMP protein complexes. Quantitative PCR demonstrated that there was no change in macrophage transcript levels of MMP-9 as a result of co-culture with MAPC (Fig. 3). The effect was specific to MAPC as co-culture of macrophages with NG2+ progenitor cells did not decrease macrophage MMP-9 secretion (Fig. 3).

The inventors also investigated the immune-modulating effects of MAPC on axonal dieback *in vivo* using an adult rat dorsal column crush model of spinal cord injury. The most dramatic phase of axonal dieback occurs between two and seven days post-lesion, which correlates spatiotemporally with the infiltration of activated macrophages into the lesion (Horn et al. 2008). The inventors hypothesized that MAPC would modulate activated macrophages and/or stimulate axonal growth within the lesion in such a way as to reduce the extent of axonal dieback and/or promote regeneration. Therefore, MAPC was transplanted into the dorsal column of the spinal cord immediately following injury and the extent of axonal dieback post-lesion was measured.

By four days post-lesion, MAPC-transplanted animals showed a significant decrease in the extent of axonal dieback compared to vehicle controls. Transplantation of MAPC significantly attenuated the extent of macrophage-mediated axonal dieback normally observed at this time. Also, MAPC-transplanted animals had decreased numbers of ED-1+ cell profiles in the lesion core at both 4 and 7 days post lesion. By seven days post-lesion, injured axons in MAPC-transplanted animals had regenerated into the lesion center, whereas axons in vehicle control treated animals did not. The inventors also observed fibers extending beyond the midpoint of the lesion at 7 days post-injury, providing further evidence of the trophic and axon growth supporting properties of this stem cell population.

### Discussion

These studies show that MAPC can positively alter the inflammatory response following injury and prevent deleterious axonal dieback. *In vivo,* MAPC could also influence axon outgrowth by altering other components of the lesion environment, such as the glial scar, through modification of astrocyte reactivity, possibly as a secondary effect of immunomodulation (Fitch et al., J Neurosci 19:8182 (1999)). Additionally, production of trophic factors, such as VEGF, by MAPC (Van't Hof et al. 2007) could be contributing to a positive outcome by enhancing cell survival or promoting the recruitment and/or proliferation of endogenous progenitor cells (Engel et al., Glia 16:16 (1996); Sasaki et al., J Neurosci 29:14932 (2009); and Thau-Zuchman et al., J Cereb Blood Flow Metab (2010)) that have been shown to provide a permissive substrate for regenerating axons (Busch et al. 2010).

These studies show that MAPCs can alter the inflammatory response to prevent, at least in part, deleterious secondary injury in the CNS. MAPC co-culture with macrophages inhibits macrophage secretion of the protease MMP-9, which the inventors have previously shown to be responsible for macrophage-induced axonal dieback (Busch et al. 2009). There are multiple stages of macrophage MMP-9 production and processing which could be impacted by MAPCs, including transcription, translation, secretion, activation, and inhibition by TIMPs. MAPC co-culture could affect macrophage MMP-9 activity at any one, or more, of these levels. However, these studies clearly demonstrate that MMP-9 transcription is not affected by MAPC co-culture, as macrophage MMP-9 mRNA levels were unchanged in the presence of MAPC compared to control conditions. The studies also indicate that activation of pro-MMP-9 is not affected by MAPC co-culture, as both the pro- and activated forms of MMP-9 protein are proportionally decreased after co-culture as seen by Western blot. It is possible that TIMP-mediated inhibition might represent a secondary level at which MAPC could further affect MMP-9 activity in dieback assays or *in vivo,* as MAPC express high levels of TIMP-1. Nevertheless, these studies clearly indicate that MAPC co-culture significantly reduced the amount of MMP-9 protein released by the cells, suggesting a direct effect on MMP-9 secretion.

While these studies have focused on evaluating the effects of MAPC on MMP-9 activity and M1/M2 activation markers, MAPC may also alter expression of other MMPs, and/or other proinflammatory molecules. Secretion of proinflammatory molecules is reflective of the general activation state of macrophages, and is decreased by shifting from an M1, or "classically activated" pro-inflammatory state, to an M2, or "alternatively activated" anti-inflammatory state (Gordon, Nat Rev Immunol 3:23 (2003)). M1 macrophages release cytokines, reactive oxygen species, nitric oxide, and MMPs, while M2 macrophages are not neurotoxic, can promote neurite outgrowth (Kigerl et al., J Neurosci 29:3435 (2009) and Block et al., Nature Reviews 8:57 (2007)), and exhibit decreased production of a number of proinflammatory molecules, including MMP-9 (Chizzolini et al, J Immunol 164:5952 (2000)). The described effects of MAPC on macrophage function may be mediated by a shift in the balance of M1/M2 activation state.

MAPC could have similar effects in other CNS injuries. The effects shown by these studies may be particularly relevant after ischemic stroke, as regulating the extent of dieback in grey matter could significantly alter the outcome after injury. MAPC could promote maintenance of cortical association as well as other types of fibers at the lesion edge, providing an enriched, synaptically connected lesion penumbra and an anatomical substrate for functional recovery.

### Methods For Figures 2-4

### DRG Dissociation

DRGs were harvested as previously described (Tom et al., J Neurosci 24:6531 (2004)). Briefly, DRGs were dissected out of adult female Sprague-Dawley rats. Both the central and peripheral roots were removed and ganglia incubated in a solution of Collagenase II (200 U/mL, Worthington) and Dispase II (2.5 U/mL, Roche) in HBSS. The digested DRGs were rinsed and gently triturated in fresh HBSS-CMF three times followed by low speed centrifugation. The dissociated DRGs were then resuspended in Neurobasal-A media supplemented with B-27, Glutamax, and Pennicillin/Streptamycin (all from Invitrogen) and counted. DRGs were plated on Delta-T dishes (Fisher) at a density of 3,000 cells/mL for a total of 6,000 cells/dish.

### Time-Lapse Dish Preparation

Delta-T cell culture dishes (Fisher) were prepared as described previously (Horn et al. 2008). Briefly, a single hole was drilled through the upper half of each dish to create a port for the addition of cells to the cultures during time-lapse microscopy. Dishes were then rinsed with sterile water and coated with poly-1-lysine (0.1 mg/mL, Invitrogen) overnight at room temperature. Dishes were then rinsed with sterile water and allowed to dry. Aggrecan gradient spots were created by pipetting 2.0 uL of aggrecan solution (2.0 mg/mL, Sigma in HBSS-CMF, Invitrogen) onto the culture surface and allowed to dry. Six spots were placed per dish. After the aggrecan spots dried completely, the entire surface of the dish was bathed in laminin solution (10 ug/mL, BTI in HBSS-CMF) for three hours at 37 degrees Celsius. The laminin bath was then removed immediately before plating of cells.

### Cell Line Macrophage Cultures

NR8383 cells (ATCC # CRL-2192), an adult Sprague-Dawley alveolar macrophage cell line, were cultured as described in previously (Yin et al., J Neurosci 23:2284 (2003)). Briefly, cells were cultured in uncoated tissue culture flasks (Corning) in F-12K media (Invitrogen) supplemented with 15% FBS (Sigma), Glutamax, Penn/Strep (Invitrogen), and sodium bicarbonate (Sigma) and fed two to three times per week. To prepare the cell line macrophages for time-lapse microscopy experiments, cells were harvested with trypsin/EDTA (Invitrogen), washed three times, and plated in uncoated tissue culture flasks at a density of 1.0 x 10⁶/mL in serum-free F-12K. Prior to use in time-lapse experiments, the cultured cell line macrophages were harvested with EDTA and a cell scraper and resuspended in Neurobasal-A with the addition of HEPES (50uM, Sigma) at a density of 2.5 x 10⁵/70ul.

### MAPC Cultures

Sprague-Dawley rat MAPCs labeled with GFP were prepared as described previously (Van't Hof et al. 2007). Cells were grown in rat MAPC media consisting of low glucose DMEM (Invitrogen), 0.4X MCDB-201 medium (Sigma), 1X ITS liquid media supplement (Sigma), 1mg/ml linoleic acid-albumin (Sigma), 100 U/ml penicillin G sodium/100 µg/ml streptomycin sulfate (Invitrogen), 100 µM 2-P-L-ascorbid acid (Sigma), 100 ng/ml EGF (Sigma), 100 ng/ml PDGF (R&D Systems), 50 nM dexamethasone (Sigma), 1000 U/ml ESGRO (Chemicon), and 2% fetal bovine serum (Hyclone). The cultures were plated on 10 ng/ml fibronectin (Invitrogen) coated 150 cm² tissue culture flasks (Corning) at an initial density of 1000 cells/cm² and subsequent replating at 200 cells/cm². The cells were maintained in 15 ml of media/flask at 37°C and 5.0% CO₂ with passaging occurring every 3-4 days using trypsin/EDTA (Invitrogen).

### MAPC-Conditioned Media

Cells were cultured as described above and conditioned media was collected after 48 hours in 50 ml conical tubes (BD Bioscience). The conditioned media was spun down at 400 x g for 5 min at 4°C and the supernatant transferred to a new 50 ml conical tube. MAPC-conditioned media was obtained as described above and concentrated 50 fold with an Amicon Microcon Ultracel YM-3 3,000 MWCO centrifugal filter (Millipore, Bedford MA).

### MAPC-Conditioned Media-Treated Macrophages

NR8383 rat macrophages were cultured as described above. One day prior to time-lapse microscopy experiments, macrophages were harvested with trypsin/EDTA (Invitrogen), washed three times, and plated in uncoated tissue culture flasks at a density of 1.0 x 10⁶/mL in serum-free F-12K. Twenty uL of the 50-fold concentrated MAPC-conditioned media were added per 1 mL of serum-free F12K media, for a final concentration of IX. Prior to use in time-lapse experiments, the cultured cell line macrophages were harvested with EDTA and a cell scraper and resuspended in Neurobasal-A with the addition of HEPES (50uM, Sigma) at a density of 2.5 x 10⁵/70ul.

### Time-Lapse Microscopy

DRG neurons were incubated at 37°C for 48 hours prior to time-lapse imaging. Neurobasal-A media with HEPES (50uM, Sigma) was added to the culture prior to transfer to a heated stage apparatus. Time-lapse images were acquired every 30 seconds for 3 hours with a Zeiss Axiovert 405M microscope using a 100x oil-immersion objective. Growth cones were chosen that extended straight into the spot rim and had characteristic dystrophic morphology for 30 minutes to observe baseline behavior before the addition of cells or conditioned media and then observed for 3 hours. For cell-addition experiments, cultured rat-derived MAPCs were harvested from tissue culture flasks, washed three times and resuspended in Neurobasal-A media. For co-culture experiments, MAPCs (100,000/dish) were added to dorsal root ganglia neuron cultures after 24 hours and incubated at 37°C for 24 additional hours before time-lapse imaging. For experiments in which MAPC-conditioned media was added to DRG cultures during time-lapse imaging, 90 uL of 50X MAPC-CM was added after 30 minutes of baseline growth cone observation. MAPC-conditioned media-treated macrophages were added to time-lapse cultures after 30 minutes of baseline imaging (500,000 cells/dish). Extension/retraction, rate of growth, turning and branching were analyzed using Metamorph software.

### Immunocytochemisty

Following time-lapse imaging, DRGs were fixed in 4% PFA and immunostained with anti-B-tubulin-type III (1:500; Sigma), anti-chondroitin sulfate (CS-56, 1:500, Sigma) and anti-GFP (1:500, Invitrogen).

### MMP-9 assays

NR8383 cells were cultured as described above, but in 24-well plates at an initial density of 100,000 cells/cm². MAPCs were co-cultured with the NR8383 cells, via Transwells (0.4 µm; Costar), at an equal plating density. Negative control wells also received Transwell inserts, with an equivalent volume of media, but no addition of MAPCs. After 24 hours, the Transwells and MAPCs were removed and discarded. NR8383 culture media was removed, and the NR8383 cells were washed 3X with PBS and re-fed with fresh media containing no FBS. After an additional 24 hours, the NR8383 conditioned media was collected for Western blot and zymogram analyses, and NR8383 total RNA was harvested for quantitative PCR analysis.

### Western Blotting

Total conditioned media from 2 replicate wells was concentrated using the described centrifugal filters to a final volume of approximately 20 µl. The total concentrated volume was loaded onto a 10% polyacrylamide gel (Bio-Rad; each gel lane represents total conditioned media concentrated from 2 replicate wells), electrophoresed for 1-2 hours at 100V, and transferred to PVDF membrane (Immobilon membrane, Millipore). Western blots were blocked in Western Blocking Solution (Sigma) for 30 minutes, then incubated in primary antibody (α-MMP-9 #AB19016, Millipore; 1:1000 dilution in blocking solution) for 2 hours at room temperature. Western blots were washed 4X in TBS, and incubated in secondary antibody (α-rabbit IgG HRP, Promega; 1:2500 dilution in blocking solution) for 1 hour at room temperature. Bound HRP-conjugated antibody was visualized using ECL chemiluminescence reagent (GE Amersham) according to manufacturer's specifications, and imaged using a ChemiDoc-It imaging system (UVP). Background subtracted band densitometry was measured and analyzed by Student's t-test for statistical significance.

### Zymography

Total conditioned media from 2 replicate wells was concentrated as described above and loaded onto a 10% gelatin zymogram gel (Bio-Rad), and electrophoresed for 1-2 hours at 100V. Zymograms were washed in 2.5% Triton X-100 for 30 minutes, and incubated overnight at 37°C in developing buffer (50mM Tris, 200mM NaCl, 5mM CaCl₂, 0.02% Brij35). Zymograms were stained for 1 hour (0.5% Coomassie G250, 30% methanol, 10% acetic acid), and de-stained (30% methanol, 10% acetic acid) until bands were visualized. Zymograms were brightfield imaged using a ChemiDoc-It imaging system. Background subtracted band densitometry was measured and analyzed by Student's t-test for statistical significance.

### Quantitative PCR

RNA was isolated from NR8383 cells using RNEasy columns (Qiagen) according to manufacturer's specifications. Rat reference RNA (Stratagene) was used as a positive control. Synthesis of cDNA was performed with M-MLV reverse transcriptase and random hexamers (Promega). Control reactions were performed without reverse transcriptase to control for genomic DNA contamination. SYBR Green quantitative PCR was performed using the following primers: MMP-9 forward - GCGCCAGCCGACTTATGT; MMP-9 reverse - AATCCTCTGCCAGCTGTGTGT; β-actin forward - AGCCCCCTCTGAACCCTAAG; β-actin reverse - CAACACAGCCTGGATGGCTAC. qPCR was performed using an ABI 7500 with 9600 emulation. The PCR conditions were 50°C x 2 min, 95°C x 10 min, and then 40 cycles of 95°C x 15 sec, 60°C x 1 min. NR8383 MMP-9 quantitation was normalized to β-actin levels, and expressed as percent of rat reference MMP-9 signal.

### Dorsal Column Crush Lesion Model

All animal procedures were performed in accordance with the guidelines and protocols of the Animal Resource Center at Case Western Reserve University. Adult female Sprague-Dawley rats 250-300g were anesthetized with inhaled isofluorane gas (2%) for all surgical procedures. A T1 laminectomy was performed to expose the dorsal aspect of the C8 spinal cord segment. A durotomy was made bilaterally 0.75 mm from midline with a 30 gauge needle. A dorsal column crush lesion was then made by inserting Dumont # 3 jeweler's forceps into the dorsal spinal cord at C8 to a depth of 1.0 mm; squeezing the forceps holding pressure for ten seconds and repeated two additional times. Completion of the lesion was verified by observation of white matter clearing. The holes in the dura were then covered with gel film. The muscle layers were sutured with 4-0 nylon suture and the skin closed with surgical staples. Upon closing of the incision, animals received Marcaine (1.0 mg/kg) subcutaneously along the incision as well as Buprenorphine (0.1 mg/kg) intramuscularly. Post-operatively, animals were kept warm with a heating lamp during recovery from anesthesia and allowed access to food and water *ad libitum.* Animals were killed at 2, 4, or 7 days post-lesion.

### Cell Transplantation

Cultured rat-derived MAPCs or primary bone marrow-derived macrophages (which had been stimulated with interferon-gamma and LPS for 24 hours) were harvested from tissue culture flasks, washed three times in HBSS-CMF and resuspended in HBSS-CMF at a density of 200,000 cells/uL. Immediately following dorsal column crush injury, 1.0 uL of the cell suspension was injected unilaterally 0.5 mm deep into the right side dorsal columns. The injection site was 0.5 mm lateral to the midline and 0.5 mm caudal to the lesion edge. The cells were injected with forty-four 23.0 nL pulses on 15 second intervals through a pulled glass pipette attached to a Nanoject II (Drummond). The glass pipette was then withdrawn from the spinal cord two minutes after the final injection. Following the transplantation, the injection site was covered with gelfilm, the muscle layers were closed with 4-0 ethicon sutures, and the skin was closed with surgical staples. Post-operatively, animals were kept warm with a heating lamp during recovery from anesthesia and allowed access to food and water *ad libitum.* Animals were killed two or four days post-lesion.

### Axon labeling

Two days before sacrifice, the dorsal columns were labeled unilaterally with Texas-Red conjugated 3000MW dextran. Briefly, the sciatic nerve of the right hindlimb was exposed and crushed three times with Dumont #3 forceps for ten seconds. 1.0 uL of 3000MW dextran-Texas-Red 10% in sterile water was the injected via a Hamilton syringe into the sciatic nerve at the crush site. The muscle layers were closed with 4-0 nylon suture and the skin with surgical staples. Upon closing of the incision, animals received Marcaine (1.0 mg/kg) subcutaneously along the incision as well as Buprenorphine (0.1 mg/kg) intramuscularly. Post-operatively, animals will be kept warm with a heating lamp during recovery from anesthesia and allowed access to food and water *ad libitum.* Animals were killed two days following labeling with an overdose of isofluorane and perfused with PBS followed by 4% PFA. Tissue was harvested and post-fixed in 4% PFA and processed for immunohistochemistry.

### Immunohistochemistry

Tissue was post-fixed in 4% PFA overnight and then submersed in 30% sucrose overnight, frozen in OTC mounting media, and cut on a cryostat into 20 µm longitudinal sections. Tissue was then stained with anti-GFAP/Alexafluor-405, anti-ED-1/Alexafluor-594 or -633, anti-GFP/Alexafluor-488, and anti-vimentin/Alexafluor-633 and imaged on a Zeiss Axiovert 510 laser-scanning confocal microscope at 10x magnification.

### Axonal Dieback Quantification

To quantify axonal dieback three consecutive sections per animal were analyzed, starting at a depth of 200 µm below the dorsal surface of the spinal cord. The lesion center was identified via characteristic GFAP and/or vimentin staining patterns and then centered using Zeiss LSM 5 Image Browser software. The distance between the ends of 5 labeled axons projecting farthest into the lesion and the lesion center was then measured. The measurements from all sections from all animals in a group were averaged to yield the average distance of dieback per time point.

### Example 2

### Effects of MultiStem® on Ischemia-Induced Gene Expression Changes

MultiStem significantly inhibits ischemia-induced markers of immune response with infarct region. qPCR analysis confirms that MultiStem administration prevents massive ischemia-induced upregulation of markers of immune response, such as MMP12 (expressed by macrophages). qPCR analysis of different brain regions demonstrates that the changes in immune marker genes are specific to the infarct region.

Neonatal Hypoxia-Ischemia Paradigm. Neonatal hypoxia-ischemia (HI) was administered to neonatal rats on postnatal day 7 (P7). P7 pups underwent permanent ligation of the CCA, followed by exposure to 8% O2, 92% N2 for 2.5 hours. Animals were administered MultiStem® (100,000 or 1 million cells) or vehicle IV 7 days after injury. Animals were sacrificed 3 days after cell infusion for tissue harvest.

qPCR Analysis. First-strand cDNA was prepared from 500 ng RNA using MMLV RT under standard conditions. SYBR Green qPCR was performed on an Applied Biosystems 7500 PCR machine. Results were normalized to β-actin, and expressed as average percent of rat reference RNA (Stratagene).

## Claims

1. An *in vitro* method for determining an effective amount of cells for administration to a subject having a condition, wherein the condition is selected from the group consisting of acute and chronic conditions in central nervous system injury selected from the group consisting of stroke; ischemic stroke, multiple sclerosis, Alzheimer's Disease, ALS, Parkinson's Disease, hypoxic-ischemia, neonatal hypoxic ischemia, and traumatic brain or spinal cord injury, the cells being multipotent adult progenitor cells (MAPCs) that are non-embryonic stem, non-germ cells that express one or more of oct4, telomerase, rex-1, or rox-1, and/or can differentiate into cell types of at least two of endodermal, ectodermal, or mesodermal germ layers, the method comprising assessing *in vitro* in a sample from the subject, following administration of the cells to the subject, a biomarker of activated macrophage, wherein the biomarker is selected from the group comprising down regulation of pro-inflammatory cytokines selected from the group consisting of TNF-α, IL-6, IL-1β and INF-γ, and up-regulation of anti-inflammatory cytokines selected from the group consisting of IL-4, IL-10 and TGF- β, wherein a down-regulation of pro-inflammatory cytokines or up-regulation of anti-inflammatory cytokines indicates that the amount of cells administered is an effective amount.

2. The method of claim 1, wherein the administered cells are allogeneic.

3. The method of any preceding claim, wherein the administered cells increase neuroprotective (M2) and/or decrease neurotoxic (M1) activation.

4. The method of any preceding claim, wherein the subject is human.

5. An *in vitro* method for constructing a cell bank, the method comprising , expanding and storing cells for future administration to a subject, the cells being multipotent adult progenitor cells (MAPCs) **characterised in that** they are non-embryonic stem, non-germ cells that express one or more of oct4, telomerase, rex-1, or rox-1 and/or can differentiate into cell types of at least two of endodermal, ectodermal, and mesodermal germ layers, wherein the cells are assayed for having the desired potency for decreasing macrophage activation state M1 wherein potency is assessed by an assay for macrophage activation state M1.

6. An *in vitro* method for drug discovery, the method comprising , exposing cells in vitro that have a desired potency for decreasing macrophage activation state M1 to an agent to assess the effect of the agent on the ability of the cells to modulate macrophage activation, the cells being multipotent adult progenitor cells (MAPCs) **characterised in that** they are non- embryonic stem, non-germ cells that express one or more of oct4, telomerase, rex-1, or rox-1 and/or can differentiate into cell types of at least two of endodermal, ectodermal, and mesodermal germ layers, wherein the cells are assayed for having the desired potency for decreasing macrophage activation state M1 wherein potency is assessed by an assay for macrophage activation state M1.

7. A method of assaying cells, *in vitro,* for having a desired potency for decreasing macrophage activation state M1, the cells being multipotent adult progenitor cells (MAPCs) **characterised in that** they are non-embryonic stem, non-germ cells that express one or more of oct4, telomerase, rex-1, or rox-1 and/or can differentiate into cell types of at least two of endodermal, ectodermal and mesodermal germ layers, wherein potency is assessed by an assay for macrophage activation state M1.

8. The method of any preceding claim wherein the cells express oct4 and/or telomerase.

9. The method of any preceding claim wherein the cells can differentiate into cell types of at least two of endodermal, ectodermal and mesodermal germ layers.

10. The method of any preceding claim wherein the cells can differentiate into cell types of all three of the endodermal, ectodermal, and mesodermal embryonic lineages.

11. The method of any preceding claim, wherein the cells are human cells.

12. The method of any preceding claim wherein the cells are derived from bone marrow, placenta, umbilical cord and cord blood, muscle, brain, liver, spinal cord, blood or skin.

13. The method of any preceding claim wherein the cells also express one or more of sox-2 and SSEA-4.

## Patentansprüche

1. *In* vitro-Verfahren zum Bestimmen einer wirksamen Menge an Zellen zur Verabreichung an einen Patienten mit einem Leiden, wobei das Leiden ausgewählt ist aus der Gruppe bestehend aus akuten und chronischen Leiden bei einer Verletzung des zentralen Nervensystems, ausgewählt aus der Gruppe bestehend aus: Schlaganfall; ischämischem Schlaganfall, multipler Sklerose, Alzheimer-Krankheit, ALS, Parkinson-Krankheit, hypoxischer Ischämie, neonataler hypoxischer Ischämie und traumatischen Hirn- oder Rückenmarksverletzungen, wobei die Zellen multipotente adulte Vorläuferzellen (MAPCs) sind, die nichtembryonale Stamm-Nichtkeimzellen sind, die Oct4, Telomerase, Rex-1 und/oder Rox-1 exprimieren, und/oder die in Zelltypen von mindestens zwei aus endodermalen, ektodermalen und mesodermalen Keimschichten differenzieren können, wobei das Verfahren das Prüfen einer Probe des Subjekts auf einen Biomarker für aktivierte Makrophagen *in vitro* nach Verabreichung der Zellen an das Subjekt umfasst, wobei der Biomarker ausgewählt ist aus der Gruppe umfassend die Herunterregulierung von proinflammatorischen Zytokinen, ausgewählt aus der Gruppe bestehend aus TNF-α, IL-6, IL-1β und INF-γ und die Hochregulierung von entzündungshemmenden Zytokinen ausgewählt aus der Gruppe bestehend aus IL-4, IL-10 und TGF-β, wobei eine Herunterregulierung von proinflammatorischen Zytokinen bzw. eine Hochregulierung von entzündungshemmenden Zytokinen anzeigt, dass die verabreichte Zellmenge eine wirksame Menge ist.

2. Verfahren nach Anspruch 1, wobei die verabreichten Zellen allogen sind.

3. Verfahren nach einem der vorhergehenden Ansprüche, wobei die verabreichten Zellen neuroprotektive (M2) Aktivierung erhöhen und/oder neurotoxische (M1) Aktivierung verringern.

4. Verfahren nach einem der vorhergehenden Ansprüche, wobei das Subjekt ein Mensch ist.

5. *In vitro*-Verfahren zum Erzeugen einer Zellbank, wobei das Verfahren das Expandieren und Lagern von Zellen zur zukünftigen Verabreichung an ein Subjekt umfasst, wobei die Zellen multipotente adulte Vorläuferzellen (MAPCs) sind, **dadurch gekennzeichnet, dass** es sich um nichtembryonale Stamm-Nichtkeimzellen handelt, die Oct4, Telomerase, Rex-1 und/oder Rox-1 exprimieren, und/oder die in Zelltypen von mindestens zwei aus endodermalen, ektodermalen und mesodermalen Keimschichten differenzieren können, wobei die Zellen dahingehend untersucht werden, ob sie die gewünschte Wirksamkeit zum Verringern des Makrophagen-Aktivierungszustands M1 aufweisen, wobei die Wirksamkeit durch einen Assay für den Makrophagen-Aktivierungszustand M1 bestimmt wird.

6. *In vitro*-Verfahren zur Wirkstoffentdeckung, wobei das Verfahren Folgendes umfasst: Aussetzen von Zellen *in vitro,* die eine gewünschte Wirksamkeit zum Verringern des Makrophagen-Aktivierungszustands M1 aufweisen, gegenüber einem Wirkstoff, zum Auswerten der Wirkung des Wirkstoffs auf die Fähigkeit der Zellen, Makrophagenaktivierung zu modulieren, wobei die Zellen multipotente adulte Vorläuferzellen (MAPCs) sind, **dadurch gekennzeichnet, dass** es sich um nichtembryonale Stamm-Nichtkeimzellen handelt, die Oct4, Telomerase, Rex-1 und/oder Rox-1 exprimieren, und/oder die in Zelltypen von mindestens zwei aus endodermalen, ektodermalen und mesodermalen Keimschichten differenzieren können, wobei die Zellen dahingehend untersucht werden, ob sie die gewünschte Wirksamkeit zum Verringern des Makrophagen-Aktivierungszustands M1 aufweisen, wobei die Wirksamkeit durch einen Assay für den Makrophagen-Aktivierungszustand M1 bestimmt wird.

7. Verfahren zum Testen von Zellen *in vitro* darauf, ob sie eine gewünschte Wirksamkeit zum Verringern des Makrophagen-Aktivierungszustands M1 haben, wobei die Zellen multipotente adulte Vorläuferzellen (MAPCs) sind, **dadurch gekennzeichnet, dass** es sich um nichtembryonale Stamm-Nichtkeimzellen handelt, die Oct4, Telomerase, Rex-1 und/oder Rox-1 exprimieren, und/oder die in Zelltypen von mindestens zwei aus endodermalen, ektodermalen und mesodermalen Keimschichten differenzieren können, wobei die Wirksamkeit durch einen Assay für den Makrophagen-Aktivierungszustand M1 bestimmt wird.

8. Verfahren nach einem der vorhergehenden Ansprüche, wobei die Zellen Oct4 und/oder Telomerase exprimieren.

9. Verfahren nach einem der vorhergehenden Ansprüche, wobei die Zellen in Zelltypen von zumindest zwei von endodermalen, ektodermalen und mesodermalen Keimschichten differenzieren können.

10. Verfahren nach einem der vorhergehenden Ansprüche, wobei die Zellen in Zelltypen aller drei von endodermalen, ektodermalen und mesodermalen embryonalen Zelllinien differenzieren können.

11. Verfahren nach einem der vorhergehenden Ansprüche, wobei die Zellen menschliche Zellen sind.

12. Verfahren nach einem der vorhergehenden Ansprüche, wobei die Zellen abgeleitet sind aus Knochenmark, Plazenta, Nabelschnur und Nabelschnurblut, Muskel, Gehirn, Leber, Rückenmark, Blut oder Haut.

13. Verfahren nach einem der vorhergehenden Ansprüche, wobei die Zellen auch sox-2 und/oder SSEA-4 exprimieren.

## Revendications

1. Procédé *in vitro* de détermination d'une quantité efficace de cellules pour administration à un sujet ayant une affection, dans lequel l'affection est choisie dans le groupe constitué d'affections aiguës et chroniques dans une lésion du système nerveux central choisie dans le groupe constitué d'un accident vasculaire cérébral ; un accident vasculaire cérébral ischémique, la sclérose en plaques, la maladie d'Alzheimer, SLA, la maladie de Parkinson, une ischémie hypoxique, une ischémie hypoxique néonatale et une lésion traumatique du cerveau ou de la moelle épinière, les cellules étant des cellules progénitrices adultes multipotentes (MAPC) qui sont des cellules souches, non germinales, non embryonnaires qui expriment l'un ou plusieurs parmi oct4, la télomérase, rex-1 ou rox-1, et/ou peuvent se différencier en types de cellules d'au moins deux des couches germinales endodermique, ectodermique ou mésodermique, le procédé comprenant l'évaluation *in vitro* dans un échantillon provenant du sujet, après l'administration des cellules au sujet, d'un biomarqueur de macrophage activé, dans lequel le biomarqueur est choisi dans le groupe comprenant la régulation négative de procytokines inflammatoires choisies dans le groupe constitué de TNF-α, IL-6, IL-1β et INF-γ, et la régulation positive d'anticytokines inflammatoires choisies dans le groupe constitué d'IL-4, IL-10 et TGF-β, dans lequel une régulation négative de procytokines inflammatoires ou une régulation positive d'anticytokines inflammatoires indique que la quantité de cellules administrée est une quantité efficace.

2. Procédé selon la revendication 1, dans lequel les cellules administrées sont allogéniques.

3. Procédé selon l'une quelconque des revendications précédentes, dans lequel les cellules administrées augmentent l'activation neuroprotectrice (M2) et/ou diminuent l'activation neurotoxique (M1).

4. Procédé selon l'une quelconque des revendications précédentes, dans lequel le sujet est humain.

5. Procédé *in vitro* de construction d'une banque de cellules, le procédé comprenant, l'expansion et le stockage de cellules pour administration future à un sujet, les cellules étant des cellules progénitrices adultes multipotentes (MAPC) **caractérisées en ce qu'**elles sont des cellules souches non embryonnaires, non germinales qui expriment l'un ou plusieurs parmi oct4, la télomérase, rex-1 ou rox-1 et/ou peuvent se différencier en types de cellules d'au moins deux des couches germinales endodermique, ectodermique et mésodermique, dans lequel les cellules sont analysées pour déterminer si elles ont l'activité souhaitée pour diminuer l'état d'activation des macrophages M1, dans lequel l'activité est évaluée par un dosage de l'état d'activation des macrophages M1.

6. Procédé *in vitro* de découverte de médicament, le procédé comprenant, l'exposition *in vitro* de cellules qui ont une activité souhaitée pour diminuer l'état d'activation des macrophages M1 à un agent pour évaluer l'effet de l'agent sur la capacité des cellules à moduler l'activation des macrophages, les cellules étant des cellules progénitrices adultes multipotentes (MAPC) **caractérisées en ce que** elles sont des cellules souches non embryonnaires, non germinales qui expriment l'un ou plusieurs parmi oct4, la télomérase, rex-1 ou rox-1 et/ou peuvent se différencier en types de cellules d'au moins deux des couches germinales endodermique, ectodermique et mésodermique, dans lequel les cellules sont analysées pour déterminer si elles ont l'activité souhaitée pour diminuer l'état d'activation des macrophages M1, dans lequel l'activité est évaluée par un dosage de l'état d'activation des macrophages M1.

7. Procédé d'analyse de cellules, *in vitro,* pour déterminer si elles ont une activité souhaitée pour diminuer l'état d'activation des macrophages M1, les cellules étant des cellules progénitrices adultes multipotentes (MAPC) **caractérisées en ce que** elles sont des cellules souches non embryonnaires, non germinales qui expriment l'un ou plusieurs parmi oct4, la télomérase, rex-1 ou rox-1 et/ou peuvent se différencier en types de cellules d'au moins deux des couches germinales endodermique, ectodermique et mésodermique, dans lequel l'activité est évaluée par un dosage de l'état d'activation des macrophages M1.

8. Procédé selon l'une quelconque des revendications précédentes dans lequel les cellules expriment oct4 et/ou la télomérase.

9. Procédé selon l'une quelconque des revendications précédentes dans lequel les cellules peuvent se différencier en types de cellules d'au moins deux des couches germinales endodermique, ectodermique et mésodermique.

10. Procédé selon l'une quelconque des revendications précédentes dans lequel les cellules peuvent se différencier en types de cellules de l'ensemble des trois lignages endodermique, ectodermique et mésodermique embryonnaires.

11. Procédé selon l'une quelconque des revendications précédentes, dans lequel les cellules sont des cellules humaines.

12. Procédé selon l'une quelconque des revendications précédentes dans lequel les cellules sont dérivées de moelle osseuse, placenta, cordon ombilical et sang ombilical, muscle, cerveau, foie, moelle épinière, sang ou peau.

13. Procédé selon l'une quelconque des revendications précédentes dans lequel les cellules expriment en outre l'un ou plusieurs de sox-2 et SSEA-4.
